(19) Europäisches Patentamt
European Patent Office
Office européen des brevets

(11) EP 4 008 714 A1

(12) EUROPEAN PATENT APPLICATION

(43) Date of publication:
**08.06.2022 Bulletin 2022/23**

(21) Application number: **20211457.5**

(22) Date of filing: **03.12.2020**

(51) International Patent Classification (IPC):
***C07D 257/02*** *(2006.01)* ***A61K 31/395*** *(2006.01)*
***A61K 31/4178*** *(2006.01)* ***A61K 31/555*** *(2006.01)*
***A61P 43/00*** *(2006.01)* ***C07D 403/06*** *(2006.01)*
***C07D 403/14*** *(2006.01)* ***C07F 7/24*** *(2006.01)*
***C07K 5/10*** *(2006.01)*

(52) Cooperative Patent Classification (CPC):
**C07D 403/14; A61P 43/00; C07D 257/02; C07D 403/06; C07K 5/0202**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Universität Zürich**
**8006 Zürich (CH)**

(72) Inventors:
- **SHOSHAN, Michal**
**8057 Zurich (CH)**
- **TAGWA, Mohammed**
**8052 Zürich (CH)**

(74) Representative: **Schulz Junghans Patentanwälte PartGmbB**
**Großbeerenstraße 71**
**10963 Berlin (DE)**

# (54) CYCLIC TETRAPEPTIDES AND METAL COMPLEXES THEREOF

(57) The present invention provides cyclic tetrapeptides consisting of alternating α- and β-amino acids and metal complexes thereof. The cyclic tetrapeptides are particularly useful for coordinating a metal selected from Pb, Cd, Hg and As. Furthermore, the invention encompasses the use of the cyclic tetrapeptides in treating a disease, particularly metal poisoning, and the use in remediation of contaminated water and soil. Also methods for detecting said metals in various substrates are provided.

SEQUENCE LISTING

<110> Universitaet Zuerich

<120> Cyclic Tetrapeptides and Metal Complexes thereof

<130> uz452ep

<160> 15

<170> PatentIn version 3.5

<210> 1
<211> 4
<212> PRT
<213> Artificial Sequence

<220>
<223> tetrapeptide

<220>
<221> MISC_FEATURE
<222> (2)..(2)
<223> beta-Alanin

<220>
<221> MISC_FEATURE
<222> (4)..(4)
<223> beta-Alanin

<400> 1

Cys Ala Cys Ala
1

<210> 2
<211> 4
<212> PRT
<213> Artificial Sequence

<220>
<223> tetrapeptide

<220>
<221> MISC_FEATURE
<222> (2)..(2)
<223> beta-Alanine

<220>
<221> MISC_FEATURE
<222> (3)..(3)
<223> D-Cysteine

<220>
<221> MISC_FEATURE
<222> (4)..(4)
<223> beta-Alanine

<400> 2

Cys Ala Cys Ala
1

<210> 3
<211> 4
<212> PRT
<213> Artificial Sequence

<220>
<223> tetrapeptide

<220>
<221> MISC_FEATURE
<222> (2)..(2)
<223> beta-Alanine

<220>
<221> MISC_FEATURE
<222> (4)..(4)
<223> beta-Alanine

<400> 3

Met Ala Met Ala
1

<210> 4
<211> 4
<212> PRT
<213> Artificial Sequence

<220>
<223> tetrapeptide

<220>
<221> MISC_FEATURE
<222> (2)..(2)
<223> beta-Alanine

<220>
<221> MISC_FEATURE
<222> (4)..(4)
<223> beta-Alanine

<400> 4

His Ala His Ala
1

<210> 5
<211> 4
<212> PRT
<213> Artificial Sequence

<220>
<223> tetrapeptide

```
<220>
<221>  MISC_FEATURE
<222>  (2)..(2)
<223>  beta-Alanine

<220>
<221>  MISC_FEATURE
<222>  (4)..(4)
<223>  beta-Alanine

<400>  5

Asp Ala Asp Ala
1


<210>  6
<211>  4
<212>  PRT
<213>  Artificial Sequence

<220>
<223>  tetrapeptide


<220>
<221>  MISC_FEATURE
<222>  (2)..(2)
<223>  beta-Alanine

<220>
<221>  MISC_FEATURE
<222>  (4)..(4)
<223>  beta-Alanine

<400>  6

Cys Ala Met Ala
1


<210>  7
<211>  4
<212>  PRT
<213>  Artificial Sequence

<220>
<223>  tetrapeptide


<220>
<221>  MISC_FEATURE
<222>  (2)..(2)
<223>  beta-Alanine

<220>
<221>  MISC_FEATURE
<222>  (4)..(4)
<223>  beta-Alanine

<400>  7

Cys Ala His Ala
```

1

<210> 8
<211> 4
<212> PRT
<213> Artificial Sequence

<220>
<223> tetrapeptide

<220>
<221> MISC_FEATURE
<222> (2)..(2)
<223> beta-Alanine

<220>
<221> MISC_FEATURE
<222> (4)..(4)
<223> beta-Alanine

<400> 8

Cys Ala Asp Ala
1

<210> 9
<211> 4
<212> PRT
<213> Artificial Sequence

<220>
<223> tetrapeptide

<220>
<221> MISC_FEATURE
<222> (2)..(2)
<223> beta-Alanine

<220>
<221> MISC_FEATURE
<222> (4)..(4)
<223> beta-Alanine

<400> 9

Cys Ala Phe Ala
1

<210> 10
<211> 4
<212> PRT
<213> Artificial Sequence

<220>
<223> tetrapeptide

<220>

<221> MISC_FEATURE
<222> (2)..(2)
<223> beta-Aspartic acid

<220>
<221> MISC_FEATURE
<222> (4)..(4)
<223> beta-Aspartic acid

<400> 10

Cys Asp Cys Asp
1

<210> 11
<211> 4
<212> PRT
<213> Artificial Sequence

<220>
<223> tetrapeptide

<220>
<221> MISC_FEATURE
<222> (2)..(2)
<223> beta-homoGlutamic acid

<220>
<221> MISC_FEATURE
<222> (4)..(4)
<223> beta-homoGlutamic acid

<400> 11

Cys Glu Cys Glu
1

<210> 12
<211> 4
<212> PRT
<213> Artificial Sequence

<220>
<223> tetrapeptide

<220>
<221> MISC_FEATURE
<222> (1)..(1)
<223> alpha-amino acid

<220>
<221> MISC_FEATURE
<222> (1)..(4)
<223> head-to-tail cyclization

<220>
<221> MISC_FEATURE
<222> (2)..(2)
<223> beta-amino acid

<220>
<221> MISC_FEATURE
<222> (3)..(3)
<223> alpha-amino acid

<220>
<221> MISC_FEATURE
<222> (4)..(4)
<223> beta-amino acid

<400> 12

Xaa Xaa Xaa Xaa
1

<210> 13
<211> 4
<212> PRT
<213> Artificial Sequence

<220>
<223> tetrapeptide

<220>
<221> MISC_FEATURE
<222> (1)..(1)
<223> beta-amino acid

<220>
<221> MISC_FEATURE
<222> (2)..(2)
<223> alpha-amino acid

<220>
<221> MISC_FEATURE
<222> (3)..(3)
<223> beta-amino acid

<220>
<221> MISC_FEATURE
<222> (4)..(4)
<223> alpha-amino acid

<400> 13

Xaa Xaa Xaa Xaa
1

<210> 14
<211> 4
<212> PRT
<213> Artificial Sequence

<220>
<223> tetrapeptide

<220>
<221> MISC_FEATURE

<222> (1)..(1)
<223> alpha-amino acid

<220>
<221> MISC_FEATURE
<222> (2)..(2)
<223> beta-amino acid

<220>
<221> MISC_FEATURE
<222> (3)..(3)
<223> alpha-amino acid

<220>
<221> MISC_FEATURE
<222> (4)..(4)
<223> beta-amino acid

<400> 14

Xaa Xaa Xaa Xaa
1

<210> 15
<211> 4
<212> PRT
<213> Artificial Sequence

<220>
<223> tetrapeptide

<220>
<221> MISC_FEATURE
<222> (1)..(1)
<223> alpha-amino acid

<220>
<221> MISC_FEATURE
<222> (1)..(4)
<223> head-to-tail cyclization

<220>
<221> MISC_FEATURE
<222> (2)..(2)
<223> beta-Alanine

<220>
<221> MISC_FEATURE
<222> (3)..(3)
<223> alpha-amino acid

<220>
<221> MISC_FEATURE
<222> (4)..(4)
<223> beta-Alanine

<400> 15

Xaa Ala Xaa Ala
1

## Description

The present invention relates to cyclic tetrapeptides and metal complexes thereof. The cyclic tetrapeptides are suitable for coordinating a metal such as Pb, As, Cd and Hg. The invention further relates to the use of the cyclic tetrapeptides in the treatment of a disease, particularly metal poisoning, and in the diagnosis of said disease. Also, methods for removing or detecting said metal by applying the cyclic tetrapeptides to a substrate such as contaminated soil or water are provided.

### Background of the Invention

Toxic metals such as lead (Pb), arsenic (As), mercury (Hg) and cadmium (Cd) can be found in contaminated soil or water where they pose a risk to the ecosystem and health of living organisms. For example, toxic metals can enter the human body via contaminated drinking water. Furthermore, metals may accumulate in crops or animals in the food chain and may thus be ingested by humans.

Lead (Pb) is a non-essential, toxic metal considered the most harmful to human health. Pb poisoning is responsible for 1 million cases of death worldwide annually. Remarkably, every third child is poisoned by Pb, while even in the United States of America, above 3% of the children are found to have dangerous Pb concentrations in their blood.

The molecular mechanisms by which Pb is toxic are diverse and include interference with both cellular processes and organ functions. Under physiological conditions, Pb is predominantly found in its cationic state as $Pb^{2+}$ that interacts with various proteins, primarily with the thiols of cysteine (Cys) and the carboxylates of aspartic (Asp) or glutamic (Glu) acids. This tight metal-binding alters the conformation of enzymes, resulting in diminished function. $Pb^{2+}$ also substitutes several essential metal ions in metalloproteins, mainly calcium (Ca) and zinc (Zn) ions, causing protein dysfunction.

After uptake, $Pb^{2+}$ is distributed to the soft tissues, with the liver and kidneys showing the highest accumulation levels. Due to its similar ionic radius as $Ca^{2+}$, $Pb^{2+}$ can also cross the blood-brain barrier, resulting in its accumulation in the brain. Finally, a significant fraction of Pb is stored in calcified tissues, and is released into the blood during pregnancy, becoming a source of exposure to the fetus while crossing the placenta.

Chelation therapy is the current treatment for Pb poisoning. It is based on administering a drug named a chelating agent (CA) that ideally should possess several essential characteristics: (a) low toxicity of the CA and the formed complex, (b) selectivity for the respective metal ion, (c) water solubility, (d) formation of an eliminable complex, and (e) ability to penetrate cells and tissues. CAs used against Pb poisoning are predominantly *ethylenediaminetetraacetic acid* (EDTA) and *dimercaptosuccinic acid* (DMSA; Figure 1).

These small-molecule drugs accomplish some of the requirements stated above. Yet, despite being the primary treatment for Pb poisoning, they suffer significant drawbacks, mainly low metal selectivity that results in essential metals being depleted from the body during treatment, which increases drug toxicity. Further, EDTA cannot cross cellular membranes, limiting its use to extracellular targets. They are also suspected of redistributing $Pb^{2+}$ ions to the brain. As a result, these chelators are only approved for medicinal use in proven and extremely high toxic metals levels. However, critically, they are not approved for use in pregnant women, and only in rare pediatrics cases, even though these segments are among the most affected population.

Based on the above-mentioned state of the art, the objective of the present invention is to provide means and methods in the treatment of metal poisoning and its diagnosis as well as to detect and remove metals from substrates such as contaminated water or soil. This objective is attained by the subject-matter of the independent claims of the present specification, with further advantageous embodiments described in the dependent claims, examples, figures and general description of this specification.

### Summary of the Invention

A first aspect of the invention relates to a compound of formula 1, particularly of formula 1a,

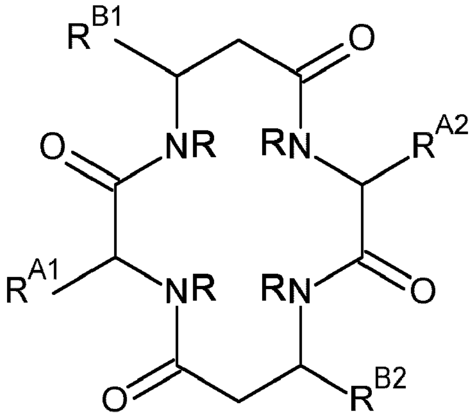

(1)

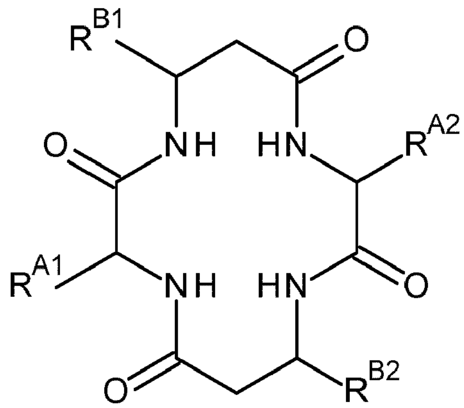

(1a),

wherein

each R independently from any other R is independently selected from $-CH_3$ and -H,

$R^{A1}$ and $R^{A2}$ are independently from each other a $C_{1-4}$-alkyl or phenyl, wherein the $C_{1-4}$-alkyl or phenyl is substituted by one or more substituents independently selected from -SH, -S-$C_{1-4}$-alkyl, -SeH, -Se-$C_{1-4}$-alkyl, $-SO_3H$, -COOH, $-NH_2$, $-CONH_2$, $-NH-C(=NH)(NH_2)$ a 5- to 10-membered heterocycle, a cyclic hydrocarbon moiety comprising 3 to 10, particularly 3 to 6, carbon atoms, wherein
the 5- to 10-membered heterocycle or the cyclic hydrocarbon moiety may optionally be substituted by one or more substituents selected from $C_{1-4}$-alkyl, - SH, (=S), -S-$C_{1-4}$-alkyl, -SeH, -Se-$C_{1-4}$-alkyl, $-SO_3H$, (=O), -COOH, $-NH_2$, -$CONH_2$,

$R^{B1}$ and $R^{B2}$ are independently form each other

- -H, or
- a moiety selected from -OH, -SH, -S-$C_{1-4}$-alkyl, -SeH, -Se-$C_{1-4}$-alkyl, -COOH, - $NH_2$, -NH-$C_{1-4}$-alkyl, -NH-$C(=NH)(NH_2)$, $-CONH_2$, $-SO_3H$, a 5- to 10-membered heterocycle or a hydrocarbon moiety comprising 1 to 12 C atoms, wherein the 5- to 10-membered heterocycle or the hydrocarbon moiety is optionally substituted by one or more substituents independently selected from -OH, (=O), -SH, (=S), -S-$C_{1-4}$-alkyl, -SeH, -Se-$C_{1-4}$-alkyl, -COOH, $-NH_2$, -NH-$C_{1-4}$-alkyl, $-NH-C(=NH)(NH_2)$, $-CONH_2$, $-SO_3H$, and a five- to 10-membered heterocycle, or
- a linker suitable for binding to a detectable marker or a solid support,
- a detectable marker, optionally linked by a linker, or
- a linker bound to a solid support.

The compound of formula 1 is a cyclic tetrapeptide consisting of two α-amino acids and two β-amino acids. The amino acids form a head-to-tail cyclization and may alternatively be represented by cyc-[Xaa-βXaa-Xaa-βXaa] (SEQ ID NO 012), wherein Xaa depicts for an α-amino acid and βXaa depicts for a β-amino acid.

The cyclic tetrapeptide is suitable for binding a metal. Discrimination between toxic metals such as $Pb^{2+}$ and other ions that are essential for human beings is achieved by a combination of the cavity size formed and the number and selection of the metal binding groups at $R^A$ and $R^B$.

$R^{A1}$ and $R^{A2}$ of the α-amino acids contribute to metal binding. Particularly for the binding of Pb, $R^{A1}$ and $R^{A2}$ each comprise a soft or borderline binding moiety. Non-limiting examples for such moieties are thiol or carboxylic acid moieties, e.g. the thiol moiety of cysteine or the β-carboxylic acid moiety of aspartic acid.

$R^{B1}$ and $R^{B2}$ of the β-amino acids may fulfil various functions such as contributing to metal binding, mediating water solubility, facilitating cyclization during synthesis and stabilizing the ring structure and the metal complex.

If β-alanine is used for βXaa, i.e. $R^B$ is H, intramolecular cyclization during synthesis is facilitated and the stability of the ring structure of the cyclic tetrapeptide is enhanced.

Water solubility of the cyclic tetrapeptide may be increased by using a moiety $R^B$ that comprises a functional

group such as an alcohol, an amide, carboxylic acid or a primary amine.

Enhancing the metal binding affinity may be achieved by additional coordination sites or by a second coordination sphere that is provided by suitable $R^B$. Also, the selectivity may be improved by steric control. For example, aliphatic or aromatic residues at $R^{B1}$ and/or $R^{B2}$ allow complex formation with smaller metal ions such as Hg.

Further functionalization of the cyclic tetrapeptide may be achieved by a linker, a linker bound to a solid support, or a detectable marker at $R^{B1}$ and/or $R^{B2}$. Such cyclic tetrapeptides may be used in the diagnosis of metal poisoning, determining the degree of contamination of a substrate such as water or soil, or in the remediation of metal contaminated soil or water.

A second aspect of the invention relates to a metal complex consisting of a ligand and a metal, wherein the ligand is a compound according to the first aspect of the invention.

As described above, the compound according to the first aspect of the invention may bind to a metal via suitable moieties at $R^A$ and $R^B$. For example, a thiol and/or carboxylic acid moiety in its deprotonated form may form a complex with $Pb^{2+}$. The metal complex may comprise only one ligand (monomeric complex) or two ligands (dimeric complex).

A third aspect of the invention relates to the use of the compound according to the first aspect of the invention in the treatment of a disease.

A fourth aspect of the invention relates to the use of the compound according to the first aspect of the invention in the treatment of metal poisoning.

In another embodiment, the present invention relates to a pharmaceutical composition comprising at least one of the compounds of the present invention or a pharmaceutically acceptable salt thereof and at least one pharmaceutically acceptable carrier, diluent or excipient.

A fifth aspect of the invention relates to a method of determining whether a patient has, or is at risk of developing metal poisoning, comprising

a. determining the level of the metal using a compound according to the first aspect of the invention in an ex vivo blood, plasma or serum sample taken from the patient, and

b. establishing the statistical significance of the concentration of the metal.

Particularly compounds according to the first aspect of the invention that comprise a detectable marker, optionally linked by a linker, at $R^B$ are suitable for the determination of the amount of metal in a sample.

A sixth aspect of the invention relates to a method of removing a metal from a substrate, wherein the method comprises using a compound according to the first aspect of the invention.

As described above, there is a constant need to remediate soil and water that are contaminated by metals such as Pb.

A seventh aspect of the invention relates to a method of detecting a metal in a substrate, wherein the method comprises using a compound according the first aspect of the invention.

Particularly compounds according to the first aspect of the invention that comprise a detectable marker, optionally linked by a linker, at $R^B$ are suitable for the detection of metals, e.g. Pb, in substrates such as contaminated water or soil.

## *Terms and definitions*

For purposes of interpreting this specification, the following definitions will apply and whenever appropriate, terms used in the singular will also include the plural and vice versa. In the event that any definition set forth below conflicts with any document incorporated herein by reference, the definition set forth shall control.

The terms "comprising," "having," "containing," and "including," and other similar forms, and grammatical equivalents thereof, as used herein, are intended to be equivalent in meaning and to be open ended in that an item or items following any one of these words is not meant to be an exhaustive listing of such item or items, or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of" or "consisting of."

Where a range of values is provided, it is understood that each intervening value, to the tenth of the unit of the lower limit, unless the context clearly dictate otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

Reference to "about" a value or parameter herein includes (and describes) variations that are directed to that

value or parameter per se. For example, description referring to "about X" includes description of "X."

As used herein, including in the appended claims, the singular forms "a," "or," and "the" include plural referents unless the context clearly dictates otherwise.

Unless defined otherwise, all technical and scientific terms used herein have the same meaning as commonly understood by one of ordinary skill in the art (e.g., in cell culture, molecular genetics, nucleic acid chemistry, hybridization techniques and biochemistry). Standard techniques are used for molecular, genetic and biochemical methods (see generally, Sambrook et al., Molecular Cloning: A Laboratory Manual, 4th ed. (2012) Cold Spring Harbor Laboratory Press, Cold Spring Harbor, N.Y. and Ausubel et al., Short Protocols in Molecular Biology (2002) 5th Ed, John Wiley & Sons, Inc.) and chemical methods.

The term "tetrapeptide" in the context of the present specification relates to a molecule consisting of 4 amino acids that form a linear chain wherein the amino acids are connected by peptide bonds. The tetrapeptide comprises two α-amino acids and two β-amino acids.

The term "cyclic tetrapeptide" relates to the tetrapeptide described above, wherein the amino acids form a head-to-tail cyclic as represented in formula 1.

Amino acid residue sequences are given from amino to carboxyl terminus. Capital letters for sequence positions refer to L-amino acids in the one-letter code (Stryer, Biochemistry, 3$^{rd}$ ed. p. 21). Lower case letters for amino acid sequence positions or "D" written before the amino acid name or amino acid code refer to the corresponding D- or (2R)-amino acids. Sequences are written left to right in the direction from the amino to the carboxy terminus. In accordance with standard nomenclature, α-amino acid residue sequences are denominated by either a three letter or a single letter code as indicated as follows: Alanine (Ala, A), Arginine (Arg, R), Asparagine (Asn, N), Aspartic Acid (Asp, D), Cysteine (Cys, C), Glutamine (Gln, Q), Glutamic Acid (Glu, E), Glycine (Gly, G), Histidine (His, H), Isoleucine (Ile, I), Leucine (Leu, L), Lysine (Lys, K), Methionine (Met, M), Phenylalanine (Phe, F), Proline (Pro, P), Serine (Ser, S), Threonine (Thr, T), Tryptophan (Trp, W), Tyrosine (Tyr, Y), and Valine (Val, V). The three letter or single letter code is also used after the Greek letter "β" for β-amino acids that comprise a residue at the β-carbon which is identical to the residue of the corresponding α-amino acid, e.g. "β-Ala" or "βAla" indicates the β-amino acid β-alanine. Homologues of α-amino acids or β-amino acids that differ by an additional methylene bridge ($-CH_2-$) in the side chain are referred to as "homo"-amino acid, e.g. homocysteine. "homo" is also abbreviated by "h", e.g. hCys depicts for the α-amino acid homocysteine and "βhGlu" depicts for β-homoglutamic acid.

In the context of the present invention, the term "5- to 10-membered heterocycle" relates to a compound that consists of 5 to 10 carbon atoms, wherein one or more carbon atoms are replaced by a heteroatom N, S or O, particularly N. Similarly, a "5- to 6-membered heterocycle" consists of 5 to 6 carbon atoms, wherein one or more carbon atoms are replaced by a heteroatom N, S or O, particularly N. The carbon atoms and one or more heteroatoms are connected by single and/or double bonds to form a ring structure. The ring structure may be monocyclic or bicyclic.

The term "hydrocarbon moiety comprising 3 to 10 carbon atoms" relates to a hydrocarbon moiety that comprises carbon-carbon single, double and/or triple bonds, particularly carbon-carbon single bonds and/or carbon-carbon double bonds. The carbon atoms may form a linear, branched or cyclic structure or combinations thereof.

The term *alkyl* refers to a linear or branched hydrocarbon moiety. A *$C_1$-$_4$-alkyl* in the context of the present specification relates to a saturated linear or branched hydrocarbon having 1, 2, 3 or 4 carbon atoms. Similarly, a $C_{1-3}$-alkyl relates to a linear or branched hydrocarbon having up to 3 carbon atoms. Non-limiting examples for a $C_1$-$C_4$ alkyl include methyl, ethyl, propyl, n-butyl, 2-methylpropyl, *tert*-butyl. In certain embodiments, a $C_{1-4}$ alkyl refers to methyl (Me), ethyl (Et), propyl (Pr), isopropyl (iPr), n-butyl (Bu) and tertbutyl (tBu).

The term *cyclic hydrocarbon moiety* relates to a mono- or polycyclic hydrocarbon moiety that comprises carbon-carbon single, double and/or triple bonds, particularly carbon-carbon single bonds and/or carbon-carbon double bonds. The ring structures of a polycyclic hydrocarbon moiety may be bridged, fused or spirocyclic. Non-limiting examples for cyclic hydrocarbon moieties are aryls, e.g. phenyl, and cycloalkyls, e.g. cyclohexyl.

The term *$C_5$-$_6$-cycloalkyl* in the context of the present specification relates to a saturated hydrocarbon ring having 5 or 6 carbon atoms.

The term *fluorescent dye* in the context of the present specification relates to a small molecule capable of fluorescence in the visible or near infrared spectrum.

## Detailed Description of the Invention

A first aspect of the invention relates to a compound of formula 1, particularly of formula 1a,

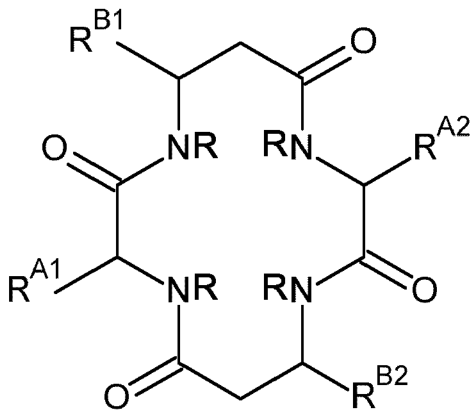

(1)

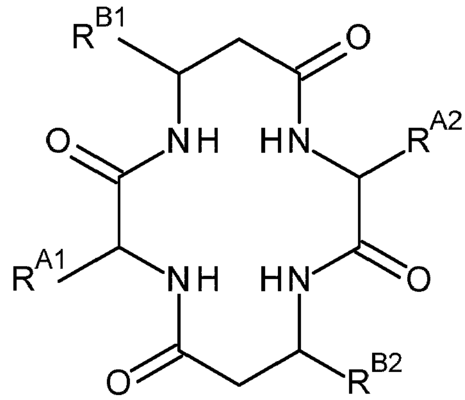

(1a),

wherein

each R independently from any other R is independently selected from $-CH_3$ and -H,

$R^{A1}$ and $R^{A2}$ are independently from each other a $C_{1-4}$-alkyl or phenyl, wherein the $C_{1-4}$-alkyl or phenyl is substituted by one or more substituents independently selected from -SH, -S-$C_{1-4}$-alkyl, -SeH, -Se-$C_{1-4}$-alkyl, $-SO_3H$, -COOH, $-NH_2$, $-CONH_2$, -NH-C(=NH)($NH_2$) a five- to 10-membered heterocycle, a cyclic hydrocarbon moiety comprising 3 to 10, particularly 3 to 6, carbon atoms, wherein
the 5- to 10-membered heterocycle or the cyclic hydrocarbon moiety may optionally be substituted by one or more substituents selected from $C_{1-4}$-alkyl, - SH, (=S), -S-$C_{1-4}$-alkyl, -SeH, -Se-$C_{1-4}$-alkyl, $-SO_3H$, (=O), -COOH, $-NH_2$, -$CONH_2$, particularly $C_{1-4}$-alkyl, -SH, -S-$C_{1-4}$-alkyl, -SeH, -Se-$C_{1-4}$-alkyl, $-SO_3H$, - COOH, $-NH_2$, $-CONH_2$,

$R^{B1}$ and $R^{B2}$ are independently form each other

- -H, or
- a moiety selected from -OH, -SH, -S-$C_{1-4}$-alkyl, -SeH, -Se-$C_{1-4}$-alkyl, -COOH, - $NH_2$, -NH-$C_{1-4}$-alkyl, -NH-C(=NH)($NH_2$), $-CONH_2$, $-SO_3H$, a five- to 10-membered heterocycle or a hydrocarbon moiety comprising 1 to 12 C atoms, wherein
the 5- to 10-membered heterocycle or the hydrocarbon moiety is optionally substituted by one or more substituents independently selected from -OH, (=O), -SH, (=S), -S-$C_{1-4}$-alkyl, -SeH, -Se-$C_{1-4}$-alkyl, -COOH, $-NH_2$, -NH-$C_{1-4}$-alkyl, -NH-C(=NH)($NH_2$), $-CONH_2$, $-SO_3H$, and a five- to 10-membered heterocycle, or
- a linker suitable for binding to a detectable marker or a solid support,
- a detectable marker, optionally linked by a linker, or
- a linker bound to a solid support.

In certain embodiments, at least one R is H and the other ones are $-CH_3$.
In certain embodiments, at least two R are H and the other ones are $-CH_3$.
In certain embodiments, at least three R are H and the other R is $-CH_3$.
In certain embodiments, at least one of the moieties $R^{A1}$ and $R^{A2}$ comprises a heteroatom S, N or O, particularly S. When the compound of formula 1 is used for binding a metal, the heteroatom at $R^A$ forms a bond to the metal such as Pb, Hg, As and Cd, particularly Pb. Binding of Pb, Hg, As and Cd, particularly Pb, may not be achieved by a hydroxyl moiety such as in the side chain of serine. Thus, $\alpha$-serine is not a suitable amino acid to provide suitable $R^A$. However, $\beta$-serine might still be used to provide a moiety $R^B$ that enhances the water solubility of the cyclic tetrapeptide.
In certain embodiments, the compound is a compound of formula 2, 3, 4, 5, 6 or 7, particularly of formula 2a, 3a, 4a, 5a, 6a or 7a,

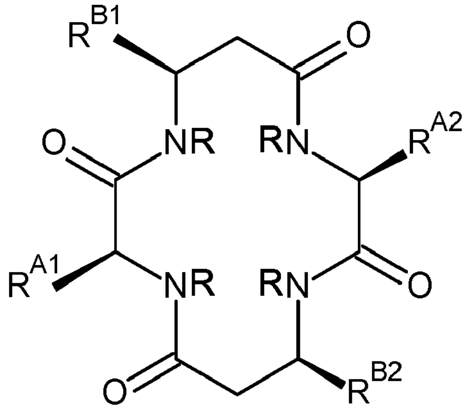

(2),

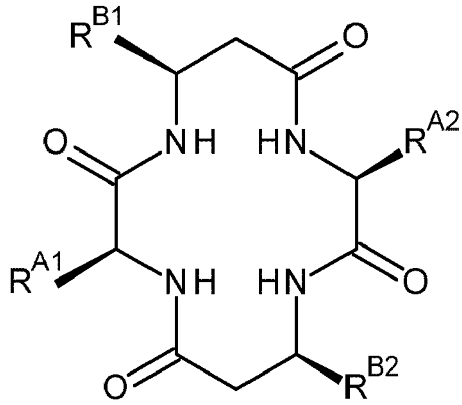

(2a),

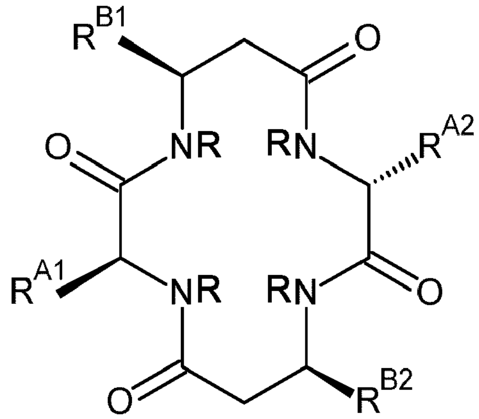

(3)

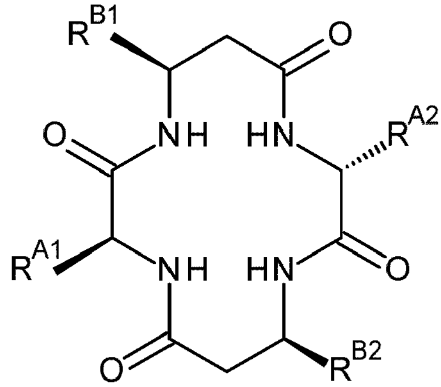

(3a),

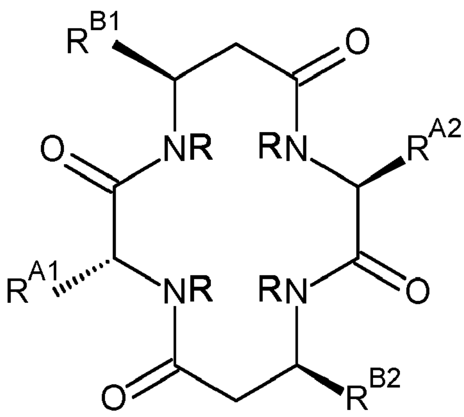

(4)

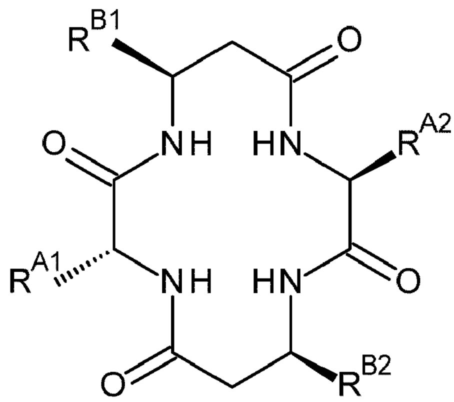

(4a),

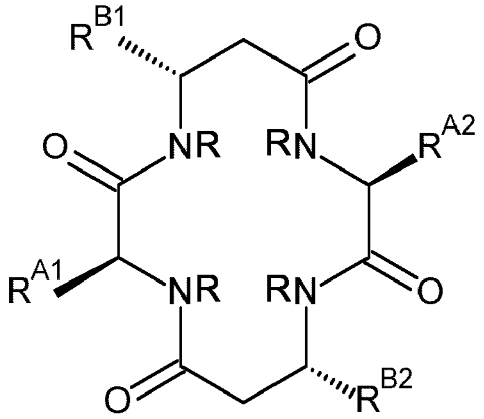

(5)

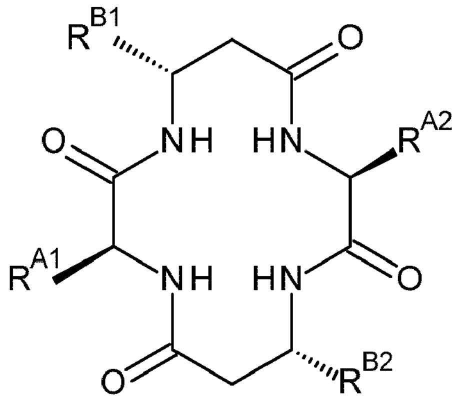

(5a),

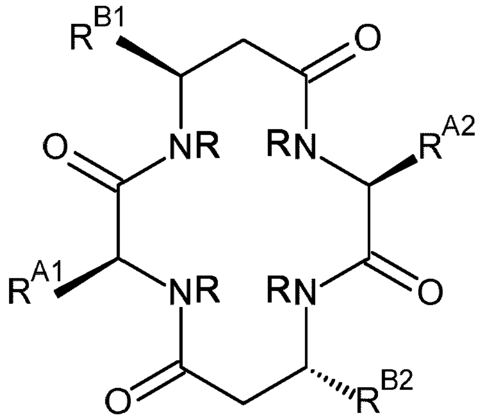

(6)

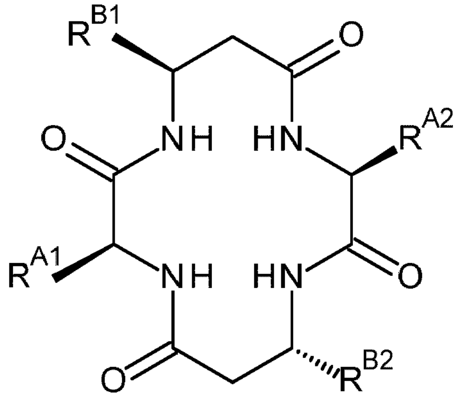

(6a),

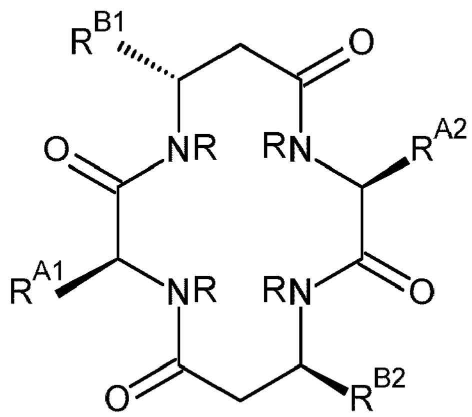

(7)

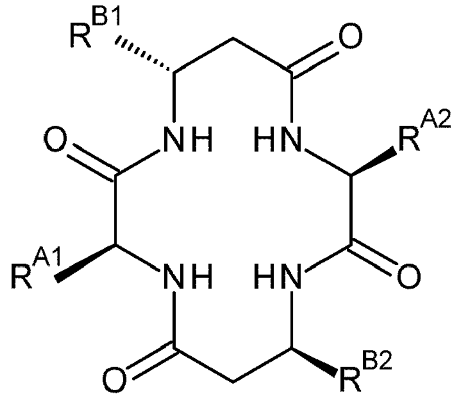

(7a).

The cyclic tetrapeptides may be formed of L- or D-amino acids or a mix thereof. Due to economic reasons, particularly L-amino acids are used since they are usually cheaper than the corresponding D-amino acids.

For stable metal complex formation, the metal binding moieties $R^{A1}$ and $R^{A2}$ should orient the same direction, particularly for capturing $Pb^{2+}$ in its favored unique hemidirected geometry.

In certain embodiments, the $\alpha$-amino acids of the cyclic tetrapeptide are both L-amino acids or are both D-amino acids, particularly are both L-amino acids. This means, $R^{A1}$ and $R^{A2}$ are both bound to the $\alpha$-carbon atom by an up-wedge bond or $R^{A1}$ and $R^{A2}$ are both bound to the $\alpha$-carbon atom by a down-wedge bond.

In certain embodiments, the compound is a compound of formula 2, 5, 6 or 7, particularly of formula 2a, 5a, 6a or 7a.

In certain embodiments, the compound is a compound of formula 2 or 5, particularly of formula 2a or 5a.

In certain embodiments, the compound is a compound of formula 2, particularly of formula 2a.

In certain embodiments, $R^{A1}$ and $R^{A2}$ are independently from each other a $C_{1-4}$-alkyl, particularly a $C_{1-3}$-alkyl, more particularly a $C_{1-2}$-alkyl, substituted by one or more, particularly 1 or 2, substituents independently selected from -SH, -S-$C_{1-4}$-alkyl, -SeH, -Se-$C_{1-4}$-alkyl, - $SO_3H$, -COOH, -$NH_2$, -$CONH_2$, a five- to 10-membered heterocycle, a cyclic hydrocarbon moiety comprising 3 to 6 carbon atoms, wherein the 5- to 10-membered heterocycle or the cyclic hydrocarbon moiety may optionally be substituted by one or more, particularly 1, substituents selected from $C_{1-4}$-alkyl, -SH, (=S), -S-$C_{1-4}$-alkyl, -SeH, -Se-$C_{1-4}$-alkyl, -$SO_3H$, (=O),-COOH, -$NH_2$, -$CONH_2$, particularly $C_{1-4}$-alkyl, -SH, -S-$C_{1-4}$-alkyl, -SeH, -Se-$C_{1-4}$-alkyl, - $SO_3H$, ,-COOH, -$NH_2$, -$CONH_2$.

In certain embodiments, $R^{A1}$ and $R^{A2}$ are independently from each other a $C_{1-4}$-alkyl, particularly a $C_{1-3}$-alkyl, more particularly a $C_{1-2}$-alkyl, substituted by one or more, particularly 1 or 2, substituents independently selected from -SH, -S-$C_{1-4}$-alkyl, -SeH, -Se-$C_{1-4}$-alkyl, - $SO_3H$, -COOH, -$NH_2$, -$CONH_2$, a five- to 10-membered heterocycle, a cyclic hydrocarbon moiety comprising 3 to 6 carbon atoms, wherein the cyclic hydrocarbon moiety may optionally be substituted by one or more, particularly 1, substituents selected from $C_{1-4}$-alkyl, -SH, -S-$C_{1-4}$-alkyl, -SeH, -Se-$C_{1-4}$-alkyl, -$SO_3H$, -COOH, -$NH_2$, -$CONH_2$.

In certain embodiments, the heterocycle at $R^{A1}$ and $R^{A2}$ is selected from piperidinyl, piperazinyl, pyridinyl, pyrimidinyl, pyrazinyl, pyridazinyl, pyrrolyl, pyrazolyl, imidazolyl, mercaptoimidazolyl, thiofuranyl, oxazolonyl, indolyl, mercaptopurinyl, benzothiophenyl, particularly imidazolyl, mercaptoimidazolyl, thiofuranyl, indolyl, more particularly, mercaptoimidazolyl.

Thiofuran is also referred to as thiophene.

Benzothiophene is also referred to as benzothiofuran.

In certain embodiments, the heterocycle at $R^{A1}$ and $R^{A2}$ is selected from piperidinyl, piperazinyl, pyridinyl, pyrimidinyl, pyrazinyl, pyridazinyl, pyrrolyl, pyrazolyl, imidazolyl, mercaptoimidazolyl, thiofuranyl, oxazolonyl.

In certain embodiments, the heterocycle at $R^{A1}$ and $R^{A2}$ is selected from pyrrolyl, pyrazolyl, imidazolyl, mercaptoimidazolyl, thiofuranyl, oxazolonyl.

In certain embodiments, the heterocycle at $R^{A1}$ and $R^{A2}$ is selected from imidazolyl, mercaptoimidazolyl, thiofuranyl.

In certain embodiments, the heterocycle at $R^{A1}$ and $R^{A2}$ is selected from pyrrolyl, pyrazolyl, imidazolyl.

In certain embodiments, the heterocycle at $R^{A1}$ and $R^{A2}$ is selected from imidazolyl, indolyl.

In certain embodiments, the heterocycle at $R^{A1}$ and $R^{A2}$ is selected from imidazolyl.

In certain embodiments, the imidazolyl is 1*H*-imidazol-4-yl. For example, $R^A$ is 1*H*-imidazol-4-yl when histidine is used as $\alpha$-amino acid.

In certain embodiments, the indolyl is 1*H*-indol-3-yl. For example, $R^A$ is 1*H*-indol-3-yl when tryptophan is used as $\alpha$-amino acid.

In certain embodiments, the cyclic hydrocarbon moiety at $R^{A1}$ and $R^{A2}$ is selected from cyclopentyl, cyclohexyl and phenyl.

In certain embodiments, the cyclic hydrocarbon moiety at $R^{A1}$ and $R^{A2}$ is phenyl.

In certain embodiments, $R^{A1}$ and $R^{A2}$ are independently from each other a $C_{1-3}$-alkyl, particularly a $C_{1-2}$-alkyl, substituted by 1 or 2 substituents independently selected from -SH, - S-$CH_3$, -SeH, -Se-$CH_3$, -$SO_3H$, -COOH, -$NH_2$, -$CONH_2$, imidazolyl, indolyl and phenyl, wherein the phenyl may optionally be substituted by one or more, particularly 1, substituents selected from -SH, and -SeH, particularly -SH.

In certain embodiments, $R^{A1}$ and $R^{A2}$ are independently from each other a $C_{1-3}$-alkyl, particularly a $C_{1-2}$-alkyl, substituted by 1 or 2 substituents independently selected from -SH, - S-$CH_3$, -SeH, -Se-$CH_3$, -$SO_3H$, -COOH, -$NH_2$, -$CONH_2$, imidazolyl, indolyl and phenyl, wherein the phenyl may optionally be substituted by one or more, particularly 1, substituents selected from -SH, and -SeH, particularly -SH.

In certain embodiments, $R^{A1}$ and $R^{A2}$ are independently selected from -$CH_2$-SH, -$(CH_2)_2$-SH, - $CH_2$-S-$CH_3$, -$(CH_2)_2$-S-$CH_3$, -CH(SH)(-$CH_2$-SH), -$CH_2$-CH(SH)(-$CH_2$-SH), -CH(SH)(-COOH), - CH(SH)-$CH_2$-COOH, -$CH_2$-CH(SH)(-COOH), -phenyl-SH, -$CH_2$-$SO_3H$, -$(CH_2)_2$-$SO_3H$ -$CH_2$-COOH, -$(CH_2)_2$-COOH, -$CH_2$-$NH_2$, -$(CH_2)_2$-$NH_2$, -$CH_2$-$CONH_2$, -$(CH_2)_2$-$CONH_2$, -$CH_2$-imidazolyl, -$CH_2$-mercaptoimidazolyl and -$CH_2$-phenyl.

In certain embodiments, $R^{A1}$ and $R^{A2}$ are independently selected from -$CH_2$-SH, -$(CH_2)_2$-SH, - $(CH_2)_2$-S-$CH_3$, -$CH_2$-CH(SH)(-$CH_2$-SH), -CH(SH)(-COOH), -phenyl-SH, -$CH_2$-$SO_3H$, -$CH_2$-COOH, -$CH_2$-$NH_2$, -$CH_2$-$CONH_2$, -$CH_2$-imidazolyl, and -$CH_2$-phenyl.

In certain embodiments, $R^{A1}$ and $R^{A2}$ are independently selected from -$CH_2$-SH, -$(CH_2)_2$-S-$CH_3$, -$CH_2$-COOH.

In certain embodiments,

- $R^{A1}$ and $R^{A2}$ are identical and selected from a $C_{1-4}$-alkyl, particularly a $C_{1-3}$-alkyl, more particularly a $C_{1-2}$-alkyl, substituted by one or more, particularly 1 or 2, substituents independently selected from -SH, -S-$C_{1-4}$-alkyl, -SeH, -Se-$C_{1-4}$-alkyl, -$SO_3H$, -COOH, - $NH_2$, -$CONH_2$, a 5- to 10-membered heterocycle, a cyclic hydrocarbon moiety comprising 3 to 6 carbon atoms, wherein

the cyclic hydrocarbon moiety is substituted by one or more substituents, particularly 1 substituent, selected from -SH, -S-$C_{1-4}$-alkyl, -SeH, -Se-$C_{1-4}$-alkyl, -$SO_3H$, -COOH, -$NH_2$, -$CONH_2$, and wherein the 5- to 10-membered heterocycle is optionally substituted by one or more substituents, particularly 1 substituent, selected from $C_{1-4}$-alkyl, -SH, (=S), -S-$C_{1-4}$-alkyl, -SeH, -Se-$C_{1-4}$-alkyl, -$SO_3H$, (=O), -COOH, -$NH_2$, -$CONH_2$, particularly $C_{1-4}$-alkyl, -SH, -S-$C_{1-4}$-alkyl, -SeH, -Se-$C_{1-4}$-alkyl, -$SO_3H$, -COOH, - $NH_2$, -$CONH_2$, and/or

- $R^{A1}$ is selected from a $C_{1-4}$-alkyl, particularly a $C_{1-3}$-alkyl, more particularly a $C_{1-2}$-alkyl, substituted by 1 or 2 substituents selected from -SH, -S-$C_{1-4}$-alkyl, -SeH, -Se-$C_{1-4}$-alkyl and -COOH, particularly -SH, -S-$C_{1-4}$-alkyl and -COOH, and
  $R^{A2}$ is selected from a $C_{1-4}$-alkyl, particularly a $C_{1-3}$-alkyl, more particularly a $C_{1-2}$-alkyl, substituted by one or more, particularly 1 or 2, substituents independently selected from -SH, -S-$C_{1-4}$-alkyl, -SeH, -Se-$C_{1-4}$-alkyl, -$SO_3H$, -COOH, -$NH_2$, -$CONH_2$, a 5- to 10-membered heterocycle, a cyclic hydrocarbon moiety comprising 3 to 6 carbon atoms, wherein
  the 5- to 10-membered heterocycle or the cyclic hydrocarbon moiety may optionally be substituted by one or more substituents, particularly 1 substituent, selected from $C_{1-4}$-alkyl, -SH, (=S), -S-$C_{1-4}$-alkyl, -SeH, -Se-$C_{1-4}$-alkyl, -$SO_3H$, (=O), -COOH, -$NH_2$, -$CONH_2$, particularly $C_{1-4}$-alkyl, -SH, -S-$C_{1-4}$-alkyl, -SeH, -Se-$C_{1-4}$-alkyl, -$SO_3H$, -COOH, -$NH_2$, -$CONH_2$,
  particularly $R^{A2}$ is selected from a $C_{1-4}$-alkyl, particularly a $C_{1-3}$-alkyl, more particularly a $C_{1-2}$-alkyl, substituted by 1 or 2 substituents, particularly 1 substituent, independently selected from -SH, -S-$C_{1-4}$-alkyl, -SeH, -Se-$C_{1-4}$-alkyl, -COOH, -$NH_2$, - $CONH_2$, a five- to 6-membered heterocycle, particularly imidazolyl, mercaptoimidazolyl or thiofuranyl, a phenyl, particularly an unsubstituted phenyl,
  wherein the phenyl may optionally be substituted by one or more substituents, particularly 1 substituent, selected from -SH, -S-$C_{1-4}$-alkyl, -SeH, -Se-$C_{1-4}$-alkyl, wherein $R^{A2}$ is selected in such a way that it differs from $R^{A1}$.

In certain embodiments,

- $R^{A1}$ and $R^{A2}$ are identical and selected from a $C_{1-3}$-alkyl, particularly a $C_{1-2}$-alkyl, substituted by 1 or 2 substituents independently selected from -SH, -S-$CH_3$, -SeH, - Se-$CH_3$, -$SO_3H$, -COOH, -$NH_2$, -$CONH_2$, imidazolyl, mercaptoimidazolyl, thiofuranyl, indolyl and phenyl, wherein the phenyl is substituted by one or more substituents, particularly 1 substituent, selected from -SH, and -SeH, particularly -SH, and/or
- $R^{A1}$ is selected from a $C_{1-3}$-alkyl, particularly a $C_{1-2}$-alkyl, substituted 1 or 2 substituents selected from -SH, -S-$C_{1-4}$-alkyl, -SeH, -Se-$C_{1-4}$-alkyl and -COOH, particularly -SH, -S-$C_{1-4}$-alkyl and -COOH, and
  $R^{A2}$ is selected from a $C_{1-3}$-alkyl, particularly a $C_{1-2}$-alkyl, substituted by 1 or 2 substituents independently selected from -SH, -S-$CH_3$, -SeH, -Se-$CH_3$, -$SO_3H$, - COOH, -$NH_2$, -$CONH_2$, imidazolyl, mercaptoimidazolyl, thiofuranyl, indolyl and phenyl,
  wherein the phenyl may optionally be substituted by one or more substituents, particularly 1 substituent, selected from -SH, and -SeH, particularly -SH, particularly $R^{A2}$ is selected from a $C_{1-3}$-alkyl, particularly a $C_{1-2}$-alkyl, substituted by 1 or 2 substituents, particularly 1 substituent, independently selected from -SH, -S-$CH_3$, -SeH, -Se-$CH_3$, -COOH, -$NH_2$, -$CONH_2$, imidazolyl and phenyl, particularly an unsubstituted phenyl or imidazoyl, wherein the phenyl may optionally be substituted by one or more substituents, particularly 1 substituent, selected from --SH, and -SeH, particularly -SH,
  wherein $R^{A2}$ is selected in such a way that it differs from $R^{A1}$.

In certain embodiments,

- $R^{A1}$ and $R^{A2}$ are identical and selected from -$CH_2$-SH, -$(CH_2)_2$-SH, -$(CH_2)_2$-S-$CH_3$, - $CH_2$-CH(SH)(-$CH_2$-SH), -CH(SH)(-COOH), -phenyl-SH, -$CH_2$-$SO_3H$, -$CH_2$-COOH and -$CH_2$-imidazolyl, and/or
- $R^{A1}$ is selected from -$CH_2$-SH and -CH(SH)(-COOH), and
  $R^{A2}$ is selected from -$CH_2$-SH, -$(CH_2)_2$-SH, -$(CH_2)_2$-S-$CH_3$, -$CH_2$-COOH, -$CH_2$-$NH_2$, - $CH_2$-$CONH_2$, -$CH_2$-imidazolyl, and -$CH_2$-phenyl,
  wherein $R^{A2}$ is selected in such a way that it differs from $R^{A1}$.

In certain embodiments, $R^{A1}$ and $R^{A2}$ are identical.

In certain embodiments, $R^{B1}$ and $R^{B2}$ are independently form each other

- -H, or
- a moiety selected from -OH, -SH, -S-$C_{1-4}$-alkyl, -SeH, -Se-$C_{1-4}$-alkyl, -COOH, - $NH_2$, -NH-$C_{1-4}$-alkyl, -NH-C(=NH)($NH_2$), -$CONH_2$, -$SO_3H$, a 5- to 10-membered heterocycle or a hydrocarbon moiety comprising 1 to 12 C

atoms, wherein
the 5- to 10-membered heterocycle or the hydrocarbon moiety is optionally substituted by one or more substituents independently selected from -OH, (=O), -SH, (=S), -S-$C_{1-4}$-alkyl, -SeH, -Se-$C_{1-4}$-alkyl, -COOH, -$NH_2$, -NH-$C_{1-4}$-alkyl, -NH-C(=NH)($NH_2$), -$CONH_2$, -$SO_3H$, and a five- to 10-membered heterocycle.

In certain embodiments, $R^{B1}$ and $R^{B2}$ are independently selected from

- -H, or
- a moiety selected from -OH, -SH, -S-$C_{1-4}$-alkyl, -SeH, -Se-$C_{1-4}$-alkyl, -COOH, - $NH_2$, -NH-$C_{1-4}$-alkyl, -NH-C(=NH)($NH_2$), -$CONH_2$, -$SO_3H$, a five- to 10-membered heterocycle or a hydrocarbon moiety comprising 1 to 12 C atoms,

wherein
the hydrocarbon moiety is optionally substituted by one or more substituents independently selected from -OH, -SH, -S-$C_{1-4}$-alkyl, -SeH, -Se-$C_{1-4}$-alkyl, - COOH, -$NH_2$, -NH-$C_{1-4}$-alkyl, -NH-C(=NH)($NH_2$), -$CONH_2$, -$SO_3H$, and a five-to 10-membered heterocycle.

To enhance the water solubility of the cyclic tetrapeptide according to the invention, one and/or both moieties $R^{B1}$ and $R^{B2}$ may comprise a hydrophilic moiety. In certain embodiments, at least one of $R^{B1}$ and $R^{B2}$ is independently selected from a moiety selected from -OH, -COOH, -$NH_2$, -$CONH_2$, -$SO_3H$, a five- to 10-membered heterocycle or a hydrocarbon moiety comprising 1 to 12 C atoms, wherein the hydrocarbon moiety is optionally substituted by one or more substituents independently selected from -OH, -COOH, -$NH_2$, -$CONH_2$, -$SO_3H$, and a five- to 10-membered heterocycle.

To enhance the metal binding affinity, $R^{B1}$ and $R^{B2}$ may comprise a moiety that provides additional coordination sites and/or a second coordination sphere. In certain embodiments, $R^{B1}$ and $R^{B2}$ are independently form each other a moiety selected from -SH, -S-$C_{1-4}$-alkyl, - SeH, -Se-$C_{1-4}$-alkyl, -COOH, -$NH_2$, -NH-$C_{1-4}$-alkyl, -NH-C(=NH)($NH_2$), -$CONH_2$, -$SO_3H$, a 5- to 10-membered heterocycle or a hydrocarbon moiety comprising 1 to 12 C atoms, wherein the 5- to 10-membered heterocycle or the hydrocarbon moiety is optionally substituted by one or more substituents independently selected from (=O), -SH, (=S), -S-$C_{1-4}$-alkyl, -SeH, -Se-$C_{1-4}$-alkyl, -COOH, -$NH_2$, -NH-$C_{1-4}$-alkyl, -NH-C(=NH)($NH_2$), -$CONH_2$, -$SO_3H$, and a five- to 10-membered heterocycle. In certain embodiments, at least one of $R^{B1}$ and $R^{B2}$ is independently selected from -SH, -S-$C_{1-4}$-alkyl, -SeH, -Se-$C_{1-4}$-alkyl, -COOH, -$NH_2$, -NH-$C_{1-4}$-alkyl, -NH-C(=NH)($NH_2$), -$CONH_2$, -$SO_3H$, a five- to 10-membered heterocycle or a hydrocarbon moiety comprising 1 to 12 C atoms, wherein the hydrocarbon moiety is optionally substituted by one or more substituents independently selected from -SH, -S-$C_{1-4}$-alkyl, -SeH, -Se-$C_{1-4}$-alkyl, - COOH, -$NH_2$, -NH-$C_{1-4}$-alkyl, -NH-C(=NH)($NH_2$), -$CONH_2$, -$SO_3H$, and a five- to 10-membered heterocycle.

In certain embodiments, $R^{B1}$ and $R^{B2}$ are independently selected from

- -H, or
- a moiety selected from -OH, -SH, -S-$C_{1-4}$-alkyl, -SeH, -Se-$C_{1-4}$-alkyl, -COOH,-$NH_2$, -NH-$C_{1-4}$-alkyl, -NH-C(=NH)($NH_2$), -$CONH_2$, -$SO_3H$, a 5- to 10-membered heterocycle, a cyclopentyl, a cyclohexyl, phenyl or a $C_{1-8}$-alkyl, particularly $C_{1-4}$-alkyl, wherein
the cyclopentyl, a cyclohexyl, phenyl or the $C_{1-8}$-alkyl, particularly $C_{1-4}$-alkyl, is optionally substituted by one or more substituents independently selected from -OH, -SH, -S-$C_{1-4}$-alkyl, -SeH, -Se-$C_{1-4}$-alkyl, -COOH, -$NH_2$, -NH-$C_{1-4}$-alkyl,-NH-C(=NH)($NH_2$), -$CONH_2$, -$SO_3H$, and a five- to 10-membered heterocycle, and wherein
the 5- to 10-membered heterocycle is optionally substituted by one or more substituents independently selected from -OH, (=O), -SH, (=S), -S-$C_{1-4}$-alkyl, - SeH, -Se-$C_{1-4}$-alkyl, -COOH, -$NH_2$, -NH-$C_{1-4}$-alkyl, -NH-C(=NH)($NH_2$), -$CONH_2$, -$SO_3H$.

In certain embodiments, the cyclopentyl, a cyclohexyl or phenyl at $R^B$ is unsubstituted.

In certain embodiments, the heterocycle at $R^{B1}$ and $R^{B2}$ is selected from piperidinyl, piperazinyl, pyridinyl, pyrimidinyl, pyrazinyl, pyridazinyl, pyrrolyl, pyrazolyl, imidazolyl, particularly imidazolyl, mercaptoimidazolyl, thiofuranyl, oxazolonyl, indolyl, mercaptopurinyl, benzothiophenyl benzimidazolyl, quinolyl, isoquinolyl, diazanaphthalenyl.

In certain embodiments, the heterocycle at $R^{B1}$ and $R^{B2}$ is selected from piperidinyl, piperazinyl, pyridinyl, pyrimidinyl, pyrazinyl, pyridazinyl, pyrrolyl, pyrazolyl, imidazolyl, particularly imidazolyl, indolyl.

In certain embodiments, the heterocycle at $R^{B1}$ and/or $R^{B2}$ is defined as described for $R^{A1}$ and $R^{A2}$. Reference is made to the respective embodiments relating to $R^{A1}$ and $R^{A2}$.

In certain embodiments, $R^{B1}$ and $R^{B2}$ are independently selected from H, -$C_{3-6}$-alkyl, particularly

$-CH_2-CH(CH_3)_2$, $-CH_2$-phenyl, -SH, $-(CH_2)_m-SH$, $-(CH_2)_m-COOH$ and $-(CH_2)_r-CONH_2$ with m and r being 0, 1, 2 or 3.

In certain embodiments, $R^{B1}$ and $R^{B2}$ are independently selected from H, -SH, $-(CH_2)_m-SH$, - $(CH_2)_m-COOH$ and $-(CH_2)_r-CONH_2$ with m and r being 0, 1, 2 or 3.

In certain embodiments, $R^{B1}$ and $R^{B2}$ are independently selected from H, $-(CH_2)_m-COOH$ and $-(CH_2)_r-CONH_2$ with m and r being 0, 1, 2 or 3.

In certain embodiments, $R^{B1}$ and $R^{B2}$ are independently selected from H, $-(CH_2)_m-COOH$ and $-CONH_2$ with m being 1, 2 or 3.

In certain embodiments, m is 1, 2 or 3.

In certain embodiments, r is 0 or 1, particularly 1.

In certain embodiments, $R^{B1}$ and $R^{B2}$ are -H.

In certain embodiments, $R^{B1}$ and $R^{B2}$ are identical.

To facilitate detection by the cyclic tetrapeptide and/or a metal complex comprising the cyclic tetrapeptide according to the invention, the cyclic tetrapeptide may comprise a detectable marker.

In certain embodiments, the detectable marker is selected from a dye, an affinity tag, a magnetic bead, and a moiety comprising a radioisotope.

Suitable dyes are for example fluorescent dyes that are known to someone of skill in the art.

For detection of the cyclic tetrapeptide via an affinity tag, commonly known tags may be used. Non-limiting examples for an affinity tag are strep-tag, glutathione-S-transferase (GST) tag, poly(His) tag.

In certain embodiments, the linker is a hydrocarbon moiety comprising up to 50 C atoms, particularly up to 20 C atoms, wherein one or more C atoms may optionally be replaced by O, S or N.

In certain embodiments, the solid support is a resin, a bead, a surface of an electrode or the bottom/wall of a reaction vessel, particularly a surface of an electrode, a resin or a bead, more particularly a resin or a bead.

The compound according to the first aspect of the invention might be bound via a linker to a reaction vessel such as a 96-well plate or to a flow through device. This would facilitate the use of the compound in diagnostic/detection methods and the use of the compound in the remediation of contaminated water and soil, respectively.

In certain embodiments, the compound according to the first aspect of the invention is a compound of formula X1 to X22, particularly of formula X1-11 or X14-22,

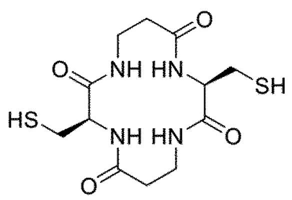 (X1),

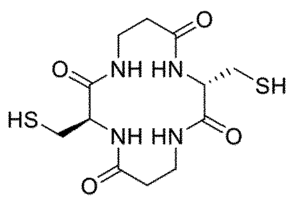 (X2),

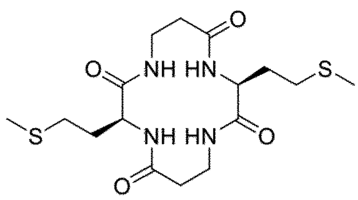 (X3),

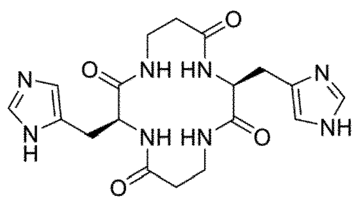 (X4),

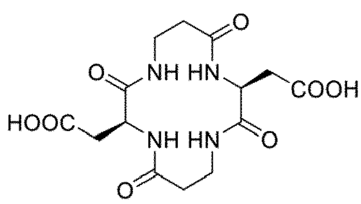 (X5),

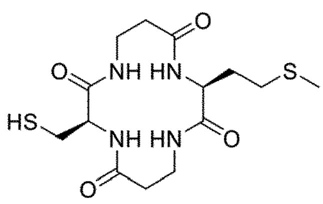 (X6),

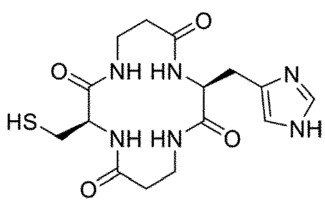 (X7),

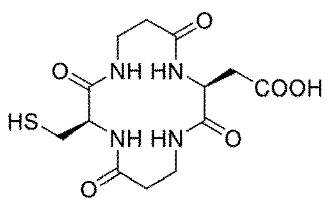 (X8),

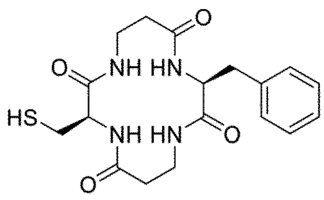 (X9), 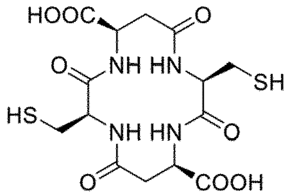 (X10), 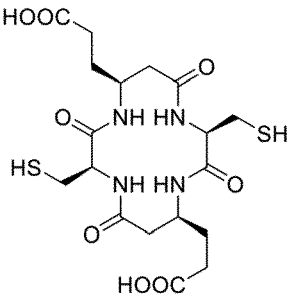 (X11),

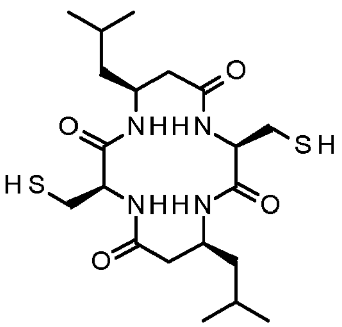 (X12), 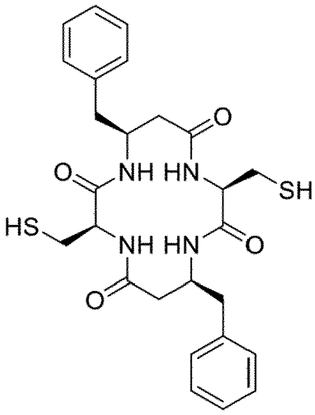 (X13),

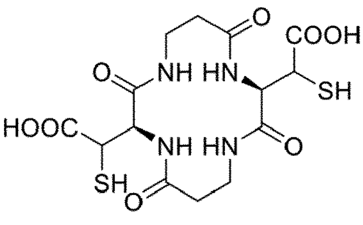 (X14), 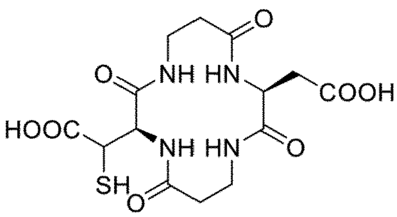 (X15),

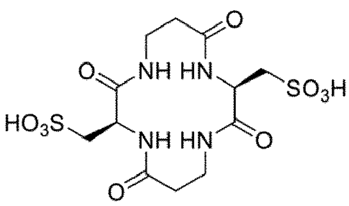 (X16),

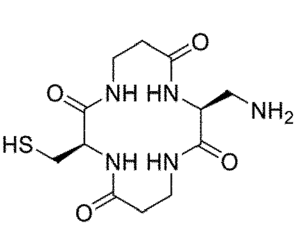 (X17), 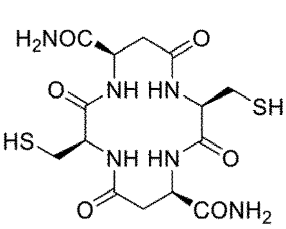 (X18), 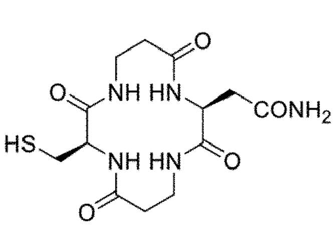 (X20),

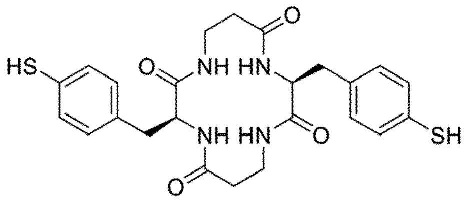
(X20),
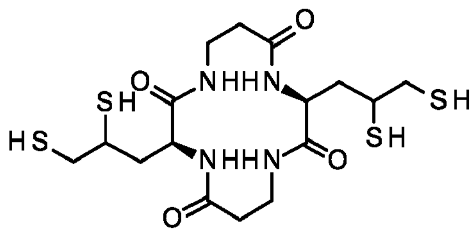
(X21),

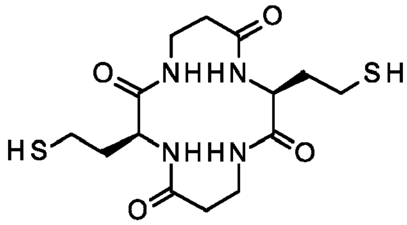
(X22).

A second aspect of the invention relates to a metal complex consisting of a ligand and a metal, wherein the ligand is a compound according to the first aspect of the invention.

As described above, the compound according to the first aspect of the invention may bind to a metal via suitable moieties at $R^A$ and $R^B$.

In certain embodiments, the binding moiety of the compound according to the first aspect of the invention binds to the metal in its deprotonated from. For example, a thiol and/or carboxylic acid moiety in its deprotonated form may form a complex with $Pb^{2+}$ as shown below (see also Fig. 3):

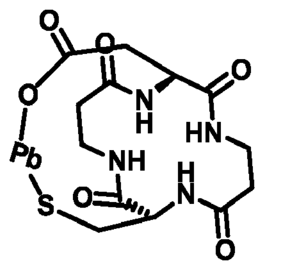
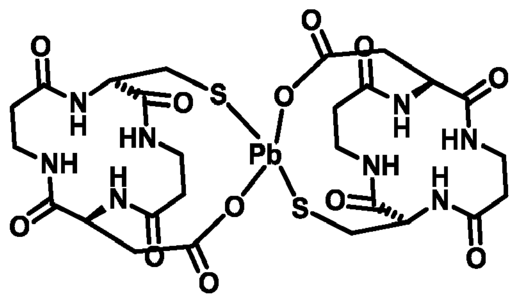
.

In certain embodiments, the ligand is an anion.

Usually the ratio of metal to peptide is 1:1 or 1:2, i.e. the complex is monomeric or dimeric.

In certain embodiments, the complex is dimeric, particularly homodimeric.

In certain embodiments, the metal is selected from Pb, As, Cd and Hg, in particular the metal is Pb.

With regard to the ligand, reference is made to the embodiments of the first aspect of the invention.

A third aspect of the invention relates to the use of the compound according to the first aspect of the invention in the treatment of a disease.

With regard to the compound, reference is made to the embodiments of the first aspect of the invention.

A fourth aspect of the invention relates to the use of the compound according to the first aspect of the invention in the treatment of metal poisoning.

In certain embodiments, the metal poisoning is selected from Pb poisoning, As poisoning, Cd poisoning and Hg poisoning.

In certain embodiments, the metal poisoning is Pb poisoning.

In a medical context, the compound according to the first aspect of the invention may be applied by standard methods as described in Sears, M.E. (2013).

With regard to the compound, reference is made to the embodiments of the first aspect of the invention.

A fifth aspect of the invention relates to a method of determining whether a patient has, or is at risk of developing metal poisoning, particularly Pb poisoning, As poisoning, Cd poisoning and Hg poisoning, more particularly Pb poisoning comprising

a. determining the level of the metal, particularly of Pb, As, Cd and/or Hg using a compound according to the first aspect of the invention in an ex vivo blood, plasma or serum sample taken from the patient, and

b. establishing the statistical significance of the concentration of the metal.

Particularly compounds according to the first aspect of the invention that comprise a detectable marker, optionally linked by a linker, at $R^B$ are suitable for the determination of the amount of metal in a sample.
Statistical significance might be established by determining the ratio of free ligand, i.e. the compound according to the first aspect of the invention, to the metal complex. The signal obtained when detecting the marker, may be compared to a standard.
With regard to the compound, reference is made to the embodiments of the first aspect of the invention.
The invention further encompasses the use of a compound according to the first aspect of the invention for use in the manufacture of a kit for the detection of developing metal poisoning, particularly Pb poisoning, As poisoning, Cd poisoning and Hg poisoning, more particularly Pb poisoning.
Wherever alternatives for single separable features are laid out herein as "embodiments", it is to be understood that such alternatives may be combined freely to form discrete embodiments of the invention disclosed herein. Thus, any of the alternative embodiments for a detectable label may be combined with any of the alternative embodiments of ligand/compound according to the first aspect of the invention and these combinations may be combined with any medical indication or diagnostic method mentioned herein.
A sixth aspect of the invention relates to a method of removing a metal, particularly a metal selected from Pb, As, Cd and Hg, more particularly Pb, from a substrate, particularly soil or an aqueous solution or aqueous suspension, wherein the method comprises using a compound according to the first aspect of the invention.
Particularly compounds according to the first aspect of the invention that comprise a detectable marker, e.g. an affinity tag, or that are bound via a linker to a solid support are suitable for this method.
With regard to the compound, reference is made to the embodiments of the first aspect of the invention.
A seventh aspect of the invention relates to a method of detecting a metal, particularly a metal selected from Pb, As, Cd and Hg, more particularly Pb, in a substrate, particularly soil or an aqueous solution or aqueous suspension, wherein the method comprises using a compound according the first aspect of the invention.
Particularly compounds according to the first aspect of the invention that comprise a detectable marker, optionally linked by a linker, at $R^B$ are suitable for the determination of the amount of metal in a sample.
With regard to the compound, reference is made to the embodiments of the first aspect of the invention.
Another aspect of the invention relates to the preparation of the compound according to the first aspect of the invention. The preparation comprises the following steps:

- Providing a tetrapeptide consisting of two α-amino acids Xaa and two β-amino acids β Xaa, wherein the tetrapeptide is characterized by the sequence βXaa-Xaa-βXaa-Xaa (SEQ ID NO 013) or Xaa-βXaa-Xaa-βXaa (SEQ ID NO 014) from N- to C-terminus, particularly βXaa-Xaa-βXaa-Xaa (SEQ ID NO 013),
- adding a coupling reagent and a base yielding a reaction mix,
- in a diluting step, diluting the reaction mix in an organic solvent, particularly $CH_2Cl_2$ or DMF, more particularly $CH_2Cl_2$.

In certain embodiments, the coupling reagent is selected from PyBOP, HATU (1-[bis(dimethylamino)methylene]-1H-1,2,3-triazolo[4,5-b]pyridinium 3-oxide hexafluorophosphate, CAS No. 148893-10-1), HCTU (*O*-(1*H*-6-chlorobenzotriazole-1-yl)-1,1,3,3-tetramethyluronium hexafluorophosphate, CAS No. 330645-87-9), HOBt/DIC (benzotriazol-1-ol, CAS No. 2592-95-2) and *N,N'*-di(propan-2-yl)methanediimine, CAS No. 693-13-0), DCC (*N,N'*-dicyclohexylmethanediimine, CAS No. 538-75-0), DPPA (diphenyl phosphorazidate, CAS No. 26386-88-9).
In certain embodiments, the coupling reagent is PyBOP. The term "PyBOP" relates to benzotriazol-1-yl-oxytripyrrolidinophosphonium hexafluorophosphate (CAS No. 128625-52-5).
In certain embodiments, 1 to 2 mole equivalents of the coupling reagent in relation to the mole amount of tetrapeptide are used.
In certain embodiments, 1.5 mole equivalents in relation to the mole amount of tetrapeptide are used.
In certain embodiments, the base is Hünig's base. The term "Hünig's base" relates to *N*-Ethyl-*N*-(propan-2-yl)propan-2-amine (CAS No. 7087-68-5).
In certain embodiments, 2 to 6 mole equivalents of base in relation to the mole amount of tetrapeptide are used.
In certain embodiments, 3 mole equivalents of base in relation to the mole amount of tetrapeptide are used.
In certain embodiments, the concentration of the tetrapeptide in the diluting step is 0.01 mM to 10 mM, particularly 0.05 mM to 2 mM.
In certain embodiments, the concentration of the tetrapeptide in the diluting step is 0.1 mM.
In certain embodiments, the dilution step is performed for 12 h to 72 h, particularly for 16 h to 48 h.

In certain embodiments, the diluting step is followed by an evaporation step. To allow fast evaporation, a solvent with a low boiling point such as $CH_2Cl_2$ may be used. When the boiling point of the solvent, e.g. DMF, is higher, evaporation may become tedious.
In certain embodiments, the method is performed at a temperature ranging from 15 °C to 40 °C, particularly ranging from 20 °C to 25 °C. The method may be performed at ambient temperature. There is no need to heat or cool down the reaction mixture.
The tetrapeptide may comprise protecting groups. Suitable protecting groups as well as methods for deprotection are known to one of skill in the art.

*Medical treatment, Dosage Forms and Salts*

Similarly, within the scope of the present invention is a method for treating metal poisoning, particularly Pb poisoning, As poisoning, Cd poisoning and Hg poisoning, more particularly Pb poisoning, in a patient in need thereof, comprising administering to the patient a compound according to the first aspect of the invention.
Similarly, a dosage form for the prevention or treatment of metal poisoning, particularly Pb poisoning, As poisoning, Cd poisoning and Hg poisoning, more particularly Pb poisoning is provided, comprising a compound according to any of the above aspects or embodiments of the invention.
The skilled person is aware that any specifically mentioned drug compound mentioned herein may be present as a pharmaceutically acceptable salt of said drug. Pharmaceutically acceptable salts comprise the ionized drug and an oppositely charged counterion. Non-limiting examples of pharmaceutically acceptable anionic salt forms include acetate, benzoate, besylate, bitatrate, bromide, carbonate, chloride, citrate, edetate, edisylate, embonate, estolate, fumarate, gluceptate, gluconate, hydrobromide, hydrochloride, iodide, lactate, lactobionate, malate, maleate, mandelate, mesylate, methyl bromide, methyl sulfate, mucate, napsylate, nitrate, pamoate, phosphate, diphosphate, salicylate, disalicylate, stearate, succinate, sulfate, tartrate, tosylate, triethiodide and valerate. Non-limiting examples of pharmaceutically acceptable cationic salt forms include aluminium, benzathine, calcium, ethylene diamine, lysine, magnesium, meglumine, potassium, procaine, sodium, tromethamine and zinc.
Dosage forms may be for enteral administration, such as nasal, buccal, rectal, transdermal or oral administration, or as an inhalation form or suppository. Alternatively, parenteral administration may be used, such as subcutaneous, intravenous, intrahepatic or intramuscular injection forms. Optionally, a pharmaceutically acceptable carrier and/or excipient may be present.
Topical administration is also within the scope of the advantageous uses of the invention. The skilled artisan is aware of a broad range of possible recipes for providing topical formulations, as exemplified by the content of Benson and Watkinson (Eds.), Topical and Transdermal Drug Delivery: Principles and Practice (1st Edition, Wiley 2011, ISBN-13: 978-0470450291); and Guy and Handcraft: Transdermal Drug Delivery Systems: Revised and Expanded (2nd Ed., CRC Press 2002, ISBN-13: 978-0824708610); Osborne and Amann (Eds.): Topical Drug Delivery Formulations (1st Ed. CRC Press 1989; ISBN-13: 978-0824781835).

*Pharmaceutical Compositions and Administration*

Another aspect of the invention relates to a pharmaceutical composition comprising a compound of the present invention, or a pharmaceutically acceptable salt thereof, and a pharmaceutically acceptable carrier. In further embodiments, the composition comprises at least two pharmaceutically acceptable carriers, such as those described herein.
In certain embodiments of the invention, the compound of the present invention is typically formulated into pharmaceutical dosage forms to provide an easily controllable dosage of the drug and to give the patient an elegant and easily handleable product.
In embodiments of the invention relating to topical uses of the compounds of the invention, the pharmaceutical composition is formulated in a way that is suitable for topical administration such as aqueous solutions, suspensions, ointments, creams, gels or sprayable formulations, e.g., for delivery by aerosol or the like, comprising the active ingredient together with one or more of solubilizers, stabilizers, tonicity enhancing agents, buffers and preservatives that are known to those skilled in the art.
The pharmaceutical composition can be formulated for enteral administration, particularly oral administration or rectal administration. In addition, the pharmaceutical compositions of the present invention can be made up in a solid form (including without limitation capsules, tablets, pills, granules, powders or suppositories), or in a liquid form (including without limitation solutions, suspensions or emulsions).
The pharmaceutical composition can be formulated for parenteral administration, for example by i.v. infusion, intradermal, subcutaneous or intramuscular administration.
The dosage regimen for the compounds of the present invention will vary depending upon known factors, such as the pharmacodynamic characteristics of the particular agent and its mode and route of administration; the species,

age, sex, health, medical condition, and weight of the recipient; the nature and extent of the symptoms; the kind of concurrent treatment; the frequency of treatment; the route of administration, the renal and hepatic function of the patient, and the effect desired. In certain embodiments, the compounds of the invention may be administered in a single daily dose, or the total daily dosage may be administered in divided doses of two, three, or four times daily.

In certain embodiments, the pharmaceutical composition or combination of the present invention can be in unit dosage of about 1-1000 mg of active ingredient(s) for a subject of about 50-70 kg. The therapeutically effective dosage of a compound, the pharmaceutical composition, or the combinations thereof, is dependent on the species of the subject, the body weight, age and individual condition, the disorder or disease or the severity thereof being treated. A physician, clinician or veterinarian of ordinary skill can readily determine the effective amount of each of the active ingredients necessary to prevent, treat or inhibit the progress of the disorder or disease.

The pharmaceutical compositions of the present invention can be subjected to conventional pharmaceutical operations such as sterilization and/or can contain conventional inert diluents, lubricating agents, or buffering agents, as well as adjuvants, such as preservatives, stabilizers, wetting agents, emulsifiers and buffers, etc. They may be produced by standard processes, for instance by conventional mixing, granulating, dissolving or lyophilizing processes. Many such procedures and methods for preparing pharmaceutical compositions are known in the art, see for example L. Lachman et al. The Theory and Practice of Industrial Pharmacy, 4th Ed, 2013 (ISBN 8123922892).

*Method of Manufacture and Method of Treatment according to the invention*

The invention further encompasses, as an additional aspect, the use of a compound according to the first aspect of the invention, or its pharmaceutically acceptable salt, as specified in detail above, for use in a method of manufacture of a medicament for the treatment or prevention of metal poisoning, particularly Pb poisoning, As poisoning, Cd poisoning and Hg poisoning, more particularly Pb poisoning.

Similarly, the invention encompasses methods of treatment of a patient having been diagnosed with a disease associated with metal poisoning, particularly Pb poisoning, As poisoning, Cd poisoning and Hg poisoning, more particularly Pb poisoning. This method entails administering to the patient an effective amount of compound according to the first aspect of the invention, or its pharmaceutically acceptable salt, as specified in detail herein.

The invention is further illustrated by the following examples and figures, from which further embodiments and advantages can be drawn. These examples are meant to illustrate the invention but not to limit its scope.

*Description of the Figures*

Fig. 1 shows DMSA and EDTA as the benchmark drugs against Pb poisoning.

Fig. 2 shows detoxification ability of tested peptides compared to benchmark drugs and glutathione (GSH) at the highest administrated concentration in vivo on DH5α cells at 120 mM (10 equiv.; a) and in vitro on HT-29 cells at 10 mM (5 equiv.; b), concentration-dependent detoxification ability in HT-29 cells of 8 and the two drugs (c) and of EDTA and 8 as Ca vs. Na salts (d), toxicity in HT-29 cells of 8 and the two drugs (e). Values are mean±SD of >3 repeats each performed in triplicate.

Fig. 3 shows a metal complex consisting of Pb and cyc[Cys-βAla-Asp-βAla] as monomeric ligand (a) and cyc[Cys-βAla-Asp-βAla] as dimeric ligand (b).

Examples

*Example 1: Synthesis of cyclic tetrapeptides*

For compounds described in this example, a scaffold composed of the sequence cyc-[Xaa-βAla-Xaa-βAla] (SEQ ID NO 015) (Xaa depicts for any α-AA; Scheme 1) was chosen, where in addition to enhancing stability, βAla was expected to facilitate the challenging intramolecular cyclization of the tetrapeptide.

**Scheme 1:** Cyclization of peptides.

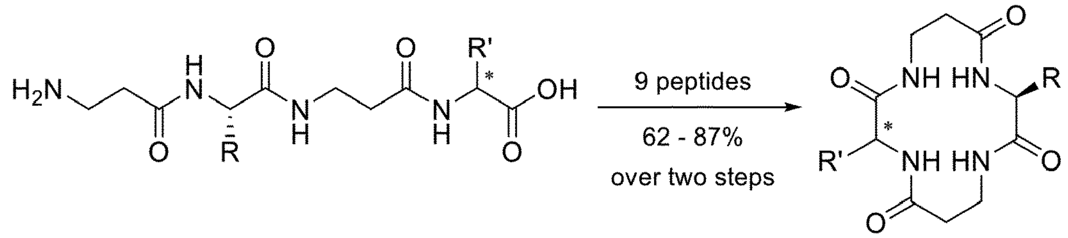


Herein the inventors present a family of cyclic tetrapeptides that were designed to detoxify $Pb^{2+}$ ions. The peptides were examined for their ability to recover Pb-exposed bacteria and human cells, where one particular peptide **(8)** excelled to a greater extent than the benchmark chelating agents (CA). Mechanistic studies of the successful peptide shed light on its biological outcome and medicinal potential.

The inventors started their studies by synthesizing nine sidechain-protected linear peptides (Table 1, 1-9). Typically, head-to-tail cyclization occurs in dimethylformamide (DMF) as a solvent and only rarely of peptides shorter than pentamers (White et al, 2011). The inventors aimed at cyclizing tetrapeptides in the absence of a high boiling-point solvent as DMF. Upon condition screening, the inventors found the following ideal conditions: PyBOP and Hünig's base (1.5 and 3.0 equivalents, respectively) as the coupling reagent and the base, respectively, and ultrahigh dilution of the peptide (0.1 mM) in $CH_2Cl_2$ for 16-48 h until full conversion was obtained. The cyclic peptides were then side-chain deprotected and purified without the need for HPLC, reaching above 95% purity and with a yield of 62-87% over two steps (cyclization and deprotection). HR-ESI-MS and $^1H$ and $^{13}C$ NMR indicated exclusively intramolecular cyclization to form the desired tetramers.

**Table 1** Peptides and benchmark compounds studied

| **Name** (SEQ ID NO) | **Sequence**[a] | **Aqueous solubility** | **Recovery**[c,d] DH5α (%) | HT-29 (%) | $IC_{50}$[e] (mM) | **Toxicity**[c,e] Maximal inhibition[f] (%) |
|---|---|---|---|---|---|---|
| 1 (SEQ ID NO 001) | [Cys-βAla]$_2$ | insoluble | 879 (164) | - | - | - |
| 2 (SEQ ID NO 002) | Cys-βAla-*D*Cys-βAla | insoluble | 436 (176) | - | - | - |
| 3 (SEQ ID NO 003) | [Met-βAla]$_2$ | insoluble | 139 (48) | - | - | - |
| 4 (SEQ ID NO 004) | [His-βAla]$_2$ | Soluble | 80 (11) | 106 (3) | 5.8 (0.7) | 62 (3) |
| 5 (SEQ ID NO 005) | [Asp-βAla]$_2$ | soluble[b] | 150 (80) | 121 (16) | *n.d.*[g] | 44 (8) |
| 6 (SEQ ID NO 006) | Cys-βAla-Met-βAla | insoluble | 403 (51) | - | - | - |
| 7 (SEQ ID NO 007) | Cys-βAla-His-βAla | insoluble | 47 (41) | - | - | - |
| 8 (SEQ ID NO 008) | Cys-βAla-Asp-βAla | soluble[b] | 279 (21) | 334 (42) | *n.d.*[g] | 15 (5) |
| 9 (SEQ ID NO 009) | Cys-βAla-Phe-βAla | insoluble | 180 (4) | - | - | - |
| 1a (SEQ ID NO 010) | [Cys-βAsp]$_2$ | soluble[b] | 2 (2) | 106 (10) | *n.d.*[g] | 0(1) |
| 1b (SEQ ID NO 011) | [Cys-βhGlu]$_2$ | soluble[b] | 30 (29) | - | - | - |
| $Na_2EDTA$ | | - | 107 (72) | 41 (6) | 0.6 (0.2) | 72 (4) |
| $Na_2CaEDTA$ | | - | 182 (39) | 95 (16) | 3.9 (0.4) | 63 (11) |
| DMSA | | - | 165 (81) | 110 (10) | 3.7 (0.1) | 77 (3) |

(continued)

| **Name** (SEQ ID NO) | **Sequence**[a] | **Aqueous solubility** | **Recovery**[c,d] DH5α (%) | **HT-29 (%)** | ***IC***$_{50}$[e] **(mM)** | **Toxicity**[c,e] **Maximal inhibition**[f] **(%)** |
|---|---|---|---|---|---|---|
| GSH[h] | | - | 39 (15) | 126 (9) | 9.6 (1.1) | 57 (8) |

*a* three-letter code of AAs, h depicts for homo; *b* with the addition of 2 equiv. NaOH or 1 equiv. of $Ca(OH)_2$; *c* values are mean±SD of >3 repeats each performed in triplicate; *d* presented values are at the highest concentrations; e the concentration at 50% viability; *f* the inhibition at the highest concentration; *g* not detected as is it too high; *h* glutathione

*Example 2: In vivo and in vitro detoxification*

Desalted peptides were then assessed for their ability to detoxify Pb (Figure 2a-d). The inventors designed two assays for rapid and reliable screening of potential CAs both *in vivo* on bacteria and then *in vitro* on human cell culture. Briefly, DH5α or HT-29 cells were first exposed to $Pb(NO_3)_2$ slightly below the minimal inhibitory concentration and then treated with various concentrations of the investigated CA, ranging from 0.1 to 10 equivalents. The viability of the cells was determined by colony counting or by crystal violet (Feoktistova et al, 2016), for the bacteria and the human cells, respectively, and was compared to poisoned cells that were not treated with any CA as the negative control. Performing both assays proved highly valuable, mainly since the *in vivo* assay examines CAs on a solidified medium, eliminating any limitations derived from low solubility of the compounds. On the other hand, testing the compounds on human cells cannot be performed with insoluble compounds but is more relevant for medicinal purposes.

Within the nine peptides, four exhibited outstanding results in detoxifying poisoned *E. coli* compared to the benchmark compounds (Figure 2a), all of which contain at least one Cys and an additional residue that can bind $Pb^{2+}$; Cys, *D*Cys, Met, and Asp for peptides **1, 2, 6,** and **8,** respectively. Treatment with **1** increased the recovery more than 8-fold while substituting one of the *L*Cys with *D*Cys reduced the detoxification ability of peptide **2.** This points to the requirement that the binding moieties should orient the same direction, capturing $Pb^{2+}$ in its favored unique hemidirected geometry. Surprisingly, the homo-functionalized peptides **3-5** showed insufficient activity, indicating a poor metal selectivity or low Pb affinity. Noteworthy, the linear analogs of peptides **1-8** were also tested *in vivo,* showing in all cases almost no detoxification ability. The inventors concluded that in addition to an expected enhanced proteolytic stability, the cyclization also fosters a preorganization of the ligand that endows its metal affinity by improving the coordination properties.

Despite being highly active *in vivo,* peptides **1, 2,** and **6** showed low aqueous solubility, reducing their effectiveness as potential CAs. Attempts to solubilize them, including in different pH conditions, formulation with PEG or co-solvent systems with DMSO failed. Therefore, two analogs of **1** where βAla is substituted by βAsp or βhGlu, to form peptides **1a** and **1b,** respectively, were synthesized. These peptides proved high solubility as Na or Ca salts, but their detoxification ability in bacteria was unsatisfied (Figure 2a). Their low activity might be related to either (a) competition in coordination with $Pb^{2+}$ by the two carboxylates, which destabilizes the complexation, or (b) a decrease in their metal selectivity and coordination alkali or alkaline earth metal ions.

Nevertheless, the inventors tested **1a** and the other soluble peptides in vitro for their ability to recover poisoned human cells (Figure 2b). Among all compounds, peptide **8** detoxified $Pb^{2+}$ to the greatest extent, with a recovery rate of 334±42%, compared to 110±10% of DMSA and 95±16% of Na2CaEDTA. This peptide dramatically excelled at high concentrations compared to the benchmark drugs and glutathione (GSH) as a natural reference peptide (Figure 2b, c). The inventors also observed similar patterns in most compounds between the two assays, suggesting that the effect of $Pb^{2+}$ on the cellular level and the chelating mechanism are similar in both systems.

The administration of EDTA as a CA has been evolved from its Na salt to $Na_2CaEDTA$, to decrease undesired depletion of $Ca^{2+}$ ions. Therefore, it was tested whether also in the case of **8,** the counter cations affect its activity (Figure 2d). Unlike EDTA, which shows a higher activity as a Ca-salt, **8** is barely affected by the counter cation (Figure 2d). These differences hint that the affinity of **8** to $Pb^{2+}$ is higher than $Ca^{2+}$ ions that, unlike with EDTA, do not bind to the CA. The lower activity of **Ca8** in high concentrations is associated with a slightly lower solubility of this salt with comparison to **$Na_2$8.**

To conclude the effectiveness of **8,** the inventors assessed its in vitro toxicity (Figure 2e) that is dramatically lower than that of DMSA and $Na_2CaEDTA$, inhibiting the viability of only 15±5% of the population.

*Material and methods*

The peptides described herein are synthesized according to the reaction shown in Scheme 2. R depicts for the side chain of an α- or β-amino acid. The side chain may be protected by a suitable protecting group (R'). The tetrapeptide

is obtained by standard solid phase peptide synthesis (SPPS) using a standard Fmoc-based protocol on a chlorotrityl chloride resin. Cleavage (1% TFA) is achieved by using TFA in $CH_2Cl_2$ in 5 rounds of 1 min each. Cyclization is obtained by reacting the sidechain-protected peptide with PyBOP (as a coupling reagent) and Hünig's base (DIPEA; as the base) in a ratio of 1.5 equivalents for PyBOP and 3 equivalents for the base, with respect to the peptide. The peptide is highly diluted (0.1 mM) to avoid dimerization and the solvent is solely $CH_2Cl_2$. The reaction mixture is incubated overnight (16-48 h). The sidechains are deprotected with a TFA cocktail that is adjusted to the respective amino acid composition. Typically, a mixture of TFA:TIPS:EDT:$H_2O$ (87.5:2.5:7.5:2.5) is applied for 1h. Finally, the cyclic tetrapeptide is purified by precipitation in an aqueous solution without the need for HPLC. Purities of 95 % and higher and yields in the range of 62 % to 87% (after purification) are reached. In a last step, the peptide is reacted with HCl so that $Cl^-$ ions replace the TFA anions as TFA is toxic. The complete removal of TFA is monitored with $^{19}F$ NMR.

**Scheme 2: Synthesis of cyclic tetrapeptides**

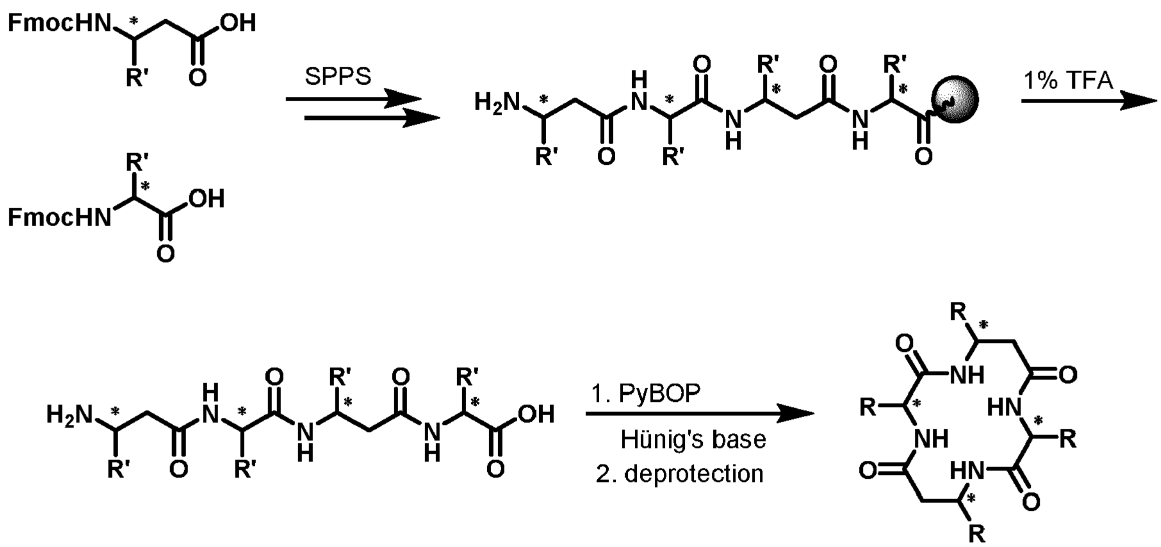


The following cyclic tetrapeptide were synthesized as described above:

**Cys-βAla-Cys-βAla** (SEQ ID NO 001)
HRMS (ESI) *m/z:* calculated for $C_{12}H_{21}N_4O_4S_2^+$ $[M+H]^+$ 349.09987; found: 349.09946
**Cys-βAla-Met-βAla** (SEQ ID 006)
HRMS (ESI) *m/z:* calculated for $C_{14}H_{25}N_4O_4S_2^+$ $[M+H]^+$ 377.13117; found: 377.13120
**His-βAla-His-βAla** (SEQ ID 004)
HRMS (ESI) *m/z:* calculated for $C_{18}H_{26}N_8O_4^{2+}$ $[M+2H]^{2+}$ 209.10330; found: 209.10341
**Cys-βAla-His-βAla** (SEQ ID 007)
HRMS (ESI) *m/z:* calculated for $C_{15}H_{23}N_6O_4S^+$ $[M+H]^+$ 383.14960; found: 383.14971
**Asp-βAla-Asp-βAla** (SEQ ID 005)
HRMS (ESI) *m/z:* calculated for $C_{14}H_{19}N_4O_8^-$ $[M-H]^-$ 371.12084; found: 371.12065
**Cys-βAla-Asp-βAla** (SEQ ID 008)
HRMS (ESI) *m/z:* calculated for $C_{13}H_{21}N_4O_6S^+$ $[M+H]^+$ 361.11763; found: 361.11771
**Cys-βAla-*D*Cys-βAla** (SEQ ID 002)
HRMS (ESI) *m/z:* calculated for $C_{12}H_{21}N_4O_4S_2^+$ $[M+H]^+$ 349.09987; found: 349.09978
**Cys-βAsp-Cys-βAsp** (SEQ ID 010)
HRMS (ESI) *m/z:* calculated for $C_{14}H_{19}N_4O_8S_2^-$ $[M-H]^-$ 435.06498; found: 435.06564
**Cys-βAla-Phe-βAla** (SEQ ID 009)
HRMS (ESI) *m/z:* calculated for $C_{18}H_{25}N_4O_4S^+$ $[M+H]^+$ 393.15910; found: 393.15888
**Met-βAla-Met-βAla** (SEQ ID 003)
HRMS (ESI) *m/z:* calculated for $C_{16}H_{28}N_4O_4SNa^+$ $[M+Na]^+$ 427.14442; found: 427.14447

***In vivo* recovery tests**

A single colony of DH5a *E. coli* WT cells was grown overnight at 37 °C and 220 rpm in Tris Minimal Medium

(TMM, pH 6.0; 5 mL) without antibiotics. The culture was then diluted to an $OD_{600}$ of 0.03 with additional TMM to a total volume of 5 mL, and its $OD_{600}$ was monitored. Upon cell density of 0.25 (which was achieved after 3-5 h), 1 mL of the culture was transferred into a cell culture tube and was labeled as the positive control. To additional 3 mL of the culture, 36 μL of $Pb(NO_3)_2$ 1 M were added (final concentration of 12 mM). Both cultures were shaken at 37 °C and 220 rpm for an additional 5 h.

Aqueous stock solutions of each CA were plated on freshly prepared agar-LB plates such that the final concentration of each compound is equal to 0.5, 1, 2, 5, and 10 equivalents, compared to the amount of $Pb(NO_3)_2$ in 50 μL pre-toxified culture. The stock solutions were prepared such that plating and equally spreading 30 μL of each solution will provide the desired amount of CA. To additional two plates, 30 μL of $H_2O$ was added.

50 μL of the pre-toxified culture were homogenously spread to each CA-containing plate, 5 h after adding the metal to the culture. The Pb-containing culture was also plated on one of the two $H_2O$-containing plates and was labeled as the negative control. Lastly, 50 μL of the non-toxified culture were platted on the second $H_2O$-containing plate and was labeled as positive control. All plates (positive and negative control and five plates for each examined compound) were then incubated at 37 °C overnight. The plates were then imaged, and the colonies were counted (with Vilber Quantum Visualization System). The recovery of each concentration of CA was calculated according to equation 1:

$$Recovery\% = \frac{\#CA_X}{\#NEG} \times 100\%$$

#CAx - number of colonies of pre-toxified culture in a plate containing X mM of CA

#NEG - number of colonies of pre-toxified culture in a plate containing no CA

Each experiment was performed on three independent occasions. Values are mean±SD of >3 repeats each performed in triplicate.

***In vitro* recovery tests**

HT-29 cells (purchased from ATCC) were grown in 25 mM HEPES RPMI-1640 medium, supplemented with 1% L-glutamine, 1% penicillin/streptomycin and 10% fetal calf serum (FCS) superior (standardized) at 37 °C and 5% $CO_2$. 96-well plates were prepared such that every well contains 10,000 cells in 100 μL medium, and the cells were allowed to adhere overnight. To all wells but the positive control, 10 μL of 22 mM $Pb(NO_3)_2$ were added (final concentration 2 mM). 10 μL of $H_2O$ was added to the positive control wells. 60 min after addition of metal, 10 μL of each solution of the examined CA (2.4, 6, 12, 24, 48, and 120 mM) were added to reach final concentrations of 0.2, 0.5, 1, 2, 4, and 10 mM (which are 0.1, 0.25, 0.5, 1, 2, and 5 equivalents, respectively). To the positive control wells containing no metal and the negative control wells containing metal but no CA, 10 μL of $H_2O$ were added. Each condition was performed in triplicates. The plates were incubated at 37 °C and 5% $CO_2$ for an additional 23 h, after which the medium was removed, washed with fresh medium and 50 μL of crystal violet solution (0.5% crystal violet powder in 20 mL MeOH and 80 mL $H_2O$) to each well and the plates were gently shaken (60 rpm) for 20 min. The plates were then washed with $H_2O$ until no more unbound dye was observed and allowed to dry overnight. 200 μL of MeOH were added to each well, and the plates were gently shaken (60 rpm) for 20 min, after which their absorbance at 560 nm was read with a plate reader. The recovery of each concentration of CA was calculated according to equation 2:

$$Recovery\% = \frac{A[CA_X] - A[blank]}{A[NEG] - A[blank]} \times 100\%$$

A[CAx] - absorbance of the pre-toxified well in the presence of X mM of CA

A[blank] - absorbance of blank wells (contain nothing)

A[NEG] - absorbance of the pre-toxified well that contains no CA

Each experiment was performed on three independent occasions. Values are mean±SD of >3 repeats each performed in triplicate.

***In vitro* toxicity tests**

HT-29 cells (purchased from ATCC) were grown in 25 mM HEPES RPMI-1640 medium, supplemented with 1% L-glutamine, 1% penicillin/streptomycin and 10% fetal calf serum (FCS) superior (standardized) at 37 °C and 5% $CO_2$. 96-well plates were prepared such that every well contains 10,000 cells in 100 μL medium, and the cells were allowed to adhere overnight. To all wells but the positive control, 10 μL of each solution of the examined CA (2.4, 6, 12, 24, 48, and 120 mM) were added to reach final concentrations of 0.2, 0.5, 1, 2, 4, and 10 mM. To the positive control wells, 10 μL of $H_2O$ was added. Each condition was performed in triplicates. The plates were incubated at 37 °C and 5% $CO_2$ for 24 h, after which the medium was removed, washed with fresh medium and 50 μL of crystal violet solution (0.5% crystal violet powder in 20 mL MeOH and 80 mL $H_2O$) to each well and the plates were gently shaken (60 rpm) for 20 min. The plates were then washed with $H_2O$ until no more unbound dye was observed and allowed to dry overnight. 200 μL of MeOH were added to each well, and the plates were gently shaken (60 rpm) for 20 min, after which their absorbance at 560 nm was read with a plate reader. The toxicity of each concentration of CA was calculated according to equation 3:

$$Toxicity\% = \frac{A[CA_X] - A[blank]}{A[POS] - A[blank]} \times 100\%$$

A[CAx] - absorbance of the pre-toxified well in the presence of X mM of CA

A[blank] - absorbance of blank wells (contain nothing)

A[POS] - absorbance of well that contains no CA

Each experiment was performed on three independent occasions. Values are mean±SD of >3 repeats each performed in triplicate.

The set-ups of in vitro and in vivo assays for determination of Pb detoxification ability could be seen in Table 1.

**Table 1.** Set-ups of *in vitro* and *in vivo* assays for determination of Pb detoxification ability

| | ***In vitro* assay** | ***In vivo* assay** |
|---|---|---|
| **Cells** | HT-29[a] | DH5α *E. coli*[b] |
| **Cell density at t=0** | 10,000 cells per well | $OD_{600}$ 0.25 |
| **$[Pb(NO_3)_2]$ (mM)** | 2[c] | 12[c] |
| **Incubated time with $Pb^{2+}$ (h)** | 1 | 5 |
| **Concentrations of compounds (mM)** | 0-10[d] | 0-120[e] |
| **Incubation with compounds** | Overnight, 37 °C, 5% $CO_2$ | Overnight, 37 °C, 220 rpm |
| **Further steps** | Washing with fresh medium | Plating on LB agar |
| **Positive control** | Non-poisoned cells | Non-poisoned cells |
| **Negative control** | Untreated cells | Untreated cells |
| **Detection of viability** | Crystal violet[f] | Colony counting[g] |

a HT-29 cells were chosen as model human cells since they show high sensitivity to Pb, and the latter does not precipitate in their medium (RPMI-1640); *b* DH5α *E. coli* strain was chosen due to its low minimal inhibitory concentration of Pb; *c* The lowest concentration that shows a significant effect at the shortest timeframe; *d* 0-5 equiv.; *e* 0-10 equiv.; *f* Dyes based on reduction (such as MTT) cannot be of use due to competing reduction by thiols; *g* Due to Pb precipitation over time, colonies counting was found to be more accurate and indicative than optical density.

List of references

Sears, Margaret E. Chelation: Harnessing and Enhancing Heavy Metal Detoxification - A Review. 2013. The Scientific World Journal, Volume 2013, Article ID 219840, 13 pages

White, C. J.; Yudin, A. K. Contemporary Strategies for Peptide Macrocyclization. Nat. Chem. 2011, 3(7), 509-524.

https://doi.org/10.1038/nchem.1062.

Feoktistova, M.; Geserick, P.; Leverkus, M. Crystal Violet Assay for Determining Viability of Cultured Cells. Cold Spring Harb. Protoc. 2016, 2016 (4), 343-346. https://doi.org/10.1101/pdb.prot087379.

**Claims**

1. A compound of formula 1, particularly of formula 1a,

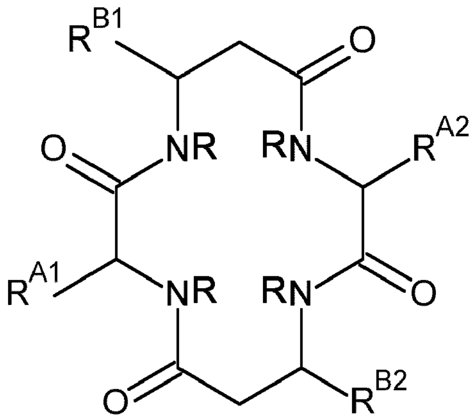
(1)

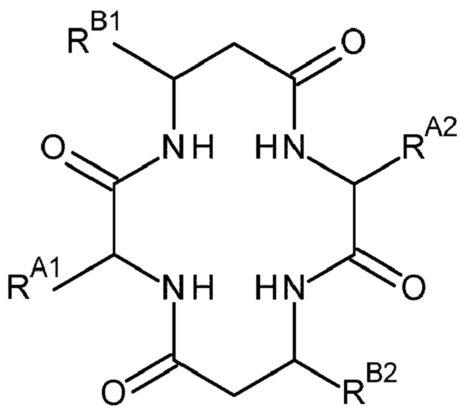
(1a),

wherein

each R independently from any other R is independently selected from $-CH_3$ and -H,
$R^{A1}$ and $R^{A2}$ are independently from each other a $C_{1-4}$-alkyl or phenyl, wherein the $C_{1-4}$-alkyl or phenyl is substituted by one or more substituents independently selected from -SH, -S-$C_{1-4}$-alkyl, -SeH, -Se-$C_{1-4}$-alkyl, $-SO_3H$, -COOH, $-NH_2$, $-CONH_2$, -NH-C(=NH)($NH_2$) a 5- to 10-membered heterocycle, a cyclic hydrocarbon moiety comprising 3 to 10, particularly 3 to 6, carbon atoms, wherein
the 5- to 10-membered heterocycle or the cyclic hydrocarbon moiety may optionally be substituted by one or more substituents selected from $C_{1-4}$-alkyl,-SH, (=S), -S-$C_{1-4}$-alkyl, -SeH, -Se-$C_{1-4}$-alkyl, $-SO_3H$, (=O), -COOH, $-NH_2$,$-CONH_2$,
$R^{B1}$ and $R^{B2}$ are independently form each other

- -H, or
- a moiety selected from -OH, -SH, -S-$C_{1-4}$-alkyl, -SeH, -Se-$C_{1-4}$-alkyl, -COOH, - $NH_2$, -NH-$C_{1-4}$-alkyl, -NH-C(=NH)($NH_2$), $-CONH_2$, $-SO_3H$, a 5- to 10-membered heterocycle or a hydrocarbon moiety comprising 1 to 12 C atoms, wherein
the 5- to 10-membered heterocycle or the hydrocarbon moiety is optionally substituted by one or more substituents independently selected from -OH, (=O), -SH, (=S), -S-$C_{1-4}$-alkyl, -SeH, -Se-$C_{1-4}$-alkyl, -COOH, $-NH_2$, -NH-$C_{1-4}$-alkyl, -NH-C(=NH)($NH_2$), $-CONH_2$, $-SO_3H$, and a five- to 10-membered heterocycle, or
- a linker suitable for binding to a detectable marker or a solid support,
- a detectable marker, optionally linked by a linker, or
- a linker bound to a solid support.

2. The compound according to claim 1, wherein the compound is a compound of formula 2, 3, 4, 5, 6 or 7 particularly of formula 2, 5, 6 or 7, more particularly of formula 2,

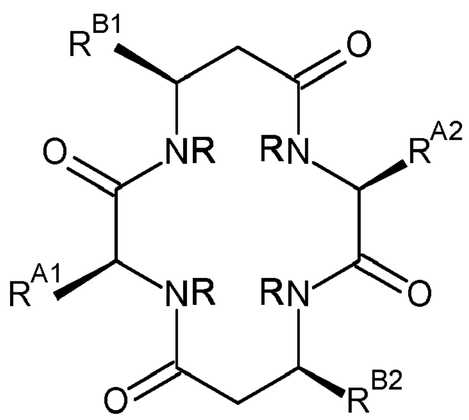

(2),

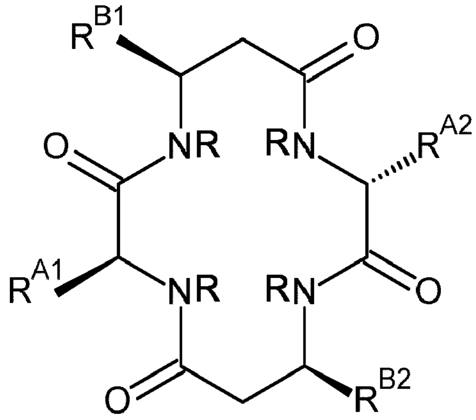

(3),

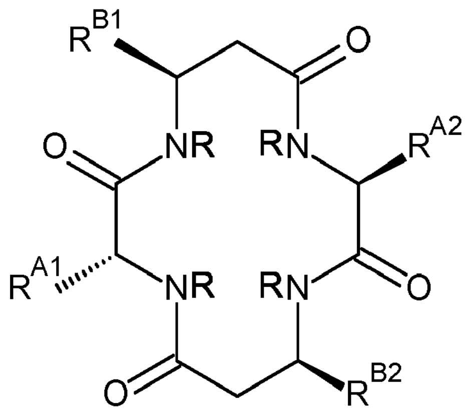

(4),

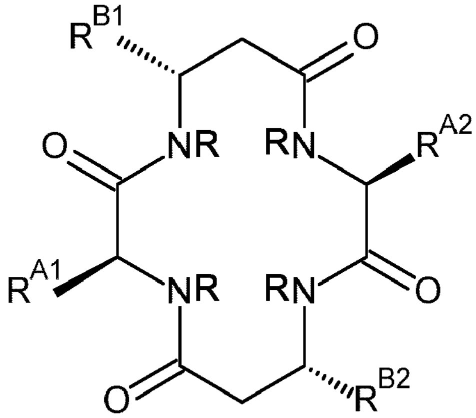

(5),

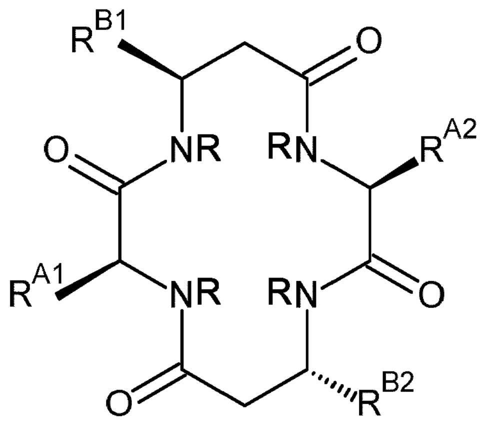

(6),

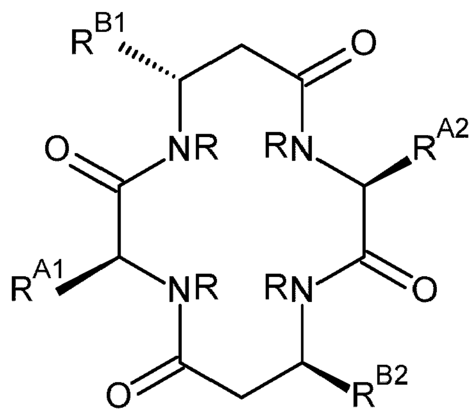

(7).

**3.** The compound according to any one of the preceding claims, wherein $R^{A1}$ and $R^{A2}$ are independently from each other a $C_{1-4}$-alkyl, particularly a $C_{1-3}$-alkyl, more particularly a $C_{1-2}$-alkyl, substituted by one or more, particularly 1 or 2, substituents independently selected from -SH, -S-$C_{1-4}$-alkyl, -SeH, -Se-$C_{1-4}$-alkyl, -$SO_3H$, -COOH, - $NH_2$, -$CONH_2$, a five- to 10-membered heterocycle, a cyclic hydrocarbon moiety comprising 3 to 6 carbon atoms, wherein the 5- to 10-membered heterocycle or the cyclic hydrocarbon moiety may optionally be substituted by one or more, particularly 1, substituents selected from $C_{1-4}$-alkyl, -SH, (=S), -S-$C_{1-4}$-alkyl, -SeH, -Se-$C_{1-4}$-alkyl, -$SO_3H$, (=O), -COOH, -$NH_2$, -$CONH_2$.

**4.** The compound according to any one of the preceding claims, wherein the cyclic hydrocarbon moiety at $R^{A1}$ and $R^{A2}$ is selected from cyclopentyl, cyclohexyl and phenyl, particularly phenyl.

**5.** The compound according to any one of the preceding claims, wherein $R^{A1}$ and $R^{A2}$ are independently from each other a $C_{1-3}$-alkyl, particularly a $C_{1-2}$-alkyl, substituted by 1 or 2 substituents independently selected from -SH, -S-$CH_3$, -SeH, -Se-$CH_3$, -$SO_3H$, -COOH, -$NH_2$, -$CONH_2$, imidazolyl, mercaptoimidazolyl, thiofuranyl, indolyl and phenyl, wherein
the phenyl may optionally be substituted by one or more, particularly 1, substituents selected from -SH, and -SeH, particularly -SH.

**6.** The compound according to any one of the preceding claims, wherein $R^{A1}$ and $R^{A2}$ are independently selected from -$CH_2$-SH, -$(CH_2)_2$-SH, -$CH_2$-S-$CH_3$, -$(CH_2)_2$-S-$CH_3$, - CH(SH)(-$CH_2$-SH), -$CH_2$-CH(SH)(-$CH_2$-SH), -CH(SH)(-COOH), -CH(SH)-$CH_2$-COOH, -$CH_2$-CH(SH)(-COOH), -phenyl-SH, -$CH_2$-$SO_3H$, -$(CH_2)_2$-$SO_3H$ -$CH_2$-COOH, -$(CH_2)_2$-COOH, -$CH_2$-$NH_2$, -$(CH_2)_2$-$NH_2$, -$CH_2$-$CONH_2$, -$(CH_2)_2$-$CONH_2$, -$CH_2$-imidazolyl, -$CH_2$-mercaptoimidazolyl and -$CH_2$-phenyl.

**7.** The compound according to any one of the preceding claims, wherein $R^{B1}$ and $R^{B2}$ are independently selected from

- -H, or
- a moiety selected from -OH, -SH, -S-$C_{1-4}$-alkyl, -SeH, -Se-$C_{1-4}$-alkyl, -COOH, - $NH_2$, -NH-$C_{1-4}$-alkyl, -NH-C(=NH)($NH_2$), -$CONH_2$, -$SO_3H$, a 5- to 10-membered heterocycle or a hydrocarbon moiety comprising 1 to 12 C atoms, wherein
the 5- to 10-membered heterocycle or the hydrocarbon moiety is optionally substituted by one or more substituents independently selected from -OH, (=O), -SH, (=S), -S-$C_{1-4}$-alkyl, -SeH, -Se-$C_{1-4}$-alkyl, -COOH, -$NH_2$, -NH-$C_{1-4}$-alkyl, -NH-C(=NH)($NH_2$), -$CONH_2$, -$SO_3H$, and a five- to 10-membered heterocycle.

**8.** The compound according to any one of the preceding claims, wherein $R^{B1}$ and $R^{B2}$ are independently selected from

- -H, or
- a moiety selected from -OH, -SH, -S-$C_{1-4}$-alkyl, -SeH, -Se-$C_{1-4}$-alkyl, -COOH, - $NH_2$, -NH-$C_{1-4}$-alkyl, -NH-C(=NH)($NH_2$), -$CONH_2$, -$SO_3H$, a 5- to 10-membered heterocycle, a cyclopentyl, a cyclohexyl, phenyl or a $C_{1-8}$-alkyl, wherein
the cyclopentyl, a cyclohexyl, phenyl or the $C_{1-8}$-alkylis optionally substituted by one or more substituents independently selected from -OH, -SH, -S-$C_{1-4}$-alkyl, -SeH, -Se-$C_{1-4}$-alkyl, -COOH, -$NH_2$, -NH-$C_{1-4}$-alkyl, -NH-C(=NH)($NH_2$), - $CONH_2$, -$SO_3H$, and a five- to 10-membered heterocycle.

**9.** The compound according to any one of the preceding claims, wherein $R^{B1}$ and $R^{B2}$ are independently selected from H, -$C_{3-6}$-alkyl, particularly -$CH_2$-CH$(CH_3)_2$, -$CH_2$-phenyl, -SH, -$(CH_2)_m$-SH, -$(CH_2)_m$-COOH and -$(CH_2)_r$-$CONH_2$ with m and r being 0, 1, 2 or 3.

**10.** The compound according to any one of the preceding claims, wherein the heterocycle at $R^{A1}$ and $R^{A2}$ and/or at $R^{B1}$ and $R^{B2}$ is selected from piperidinyl, piperazinyl, pyridinyl, pyrimidinyl, pyrazinyl, pyridazinyl, pyrrolyl, pyrazolyl, imidazolyl, particularly imidazolyl, mercaptoimidazolyl, thiofuranyl, oxazolonyl, indolyl, mercaptopurinyl, benzothiophenyl.

**11.** The compound according to any one of the preceding claims, wherein $R^{A1}$ and $R^{A2}$ are identical and/or $R^{B1}$ and $R^{B2}$ are identical.

**12.** The compound according to any one of the preceding claims, wherein
the detectable marker is selected from a dye, an affinity tag, a magnetic bead and a moiety comprising a radioisotope, and/or
the linker is a hydrocarbon moiety comprising up to 50 C atoms, wherein one or more C atoms may optionally be replaced by O, S or N, and/or
the solid support is a resin, a bead, a surface of an electrode or the bottom/wall of a reaction vessel.

**13.** A metal complex consisting of a ligand and a metal, particularly a metal selected from Pb, As, Cd and Hg, wherein the ligand is a compound according to any of claims 1 to 12.

**14.** A use of the compound according to any of claims 1 to 12 in the treatment of a disease, in particular in the treatment of metal poisoning, more particularly Pb poisoning, As poisoning, Cd poisoning and Hg poisoning.

**15.** A method of removing and/or detecting a metal, particularly a metal selected from Pb, As, Cd and Hg, more particularly Pb, from/in a substrate, particularly soil or an aqueous solution or aqueous suspension, wherein the method comprises using a compound according to any of claims 1 to 12.

Fig. 1

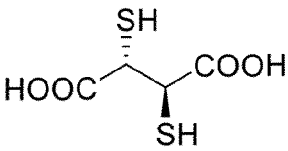

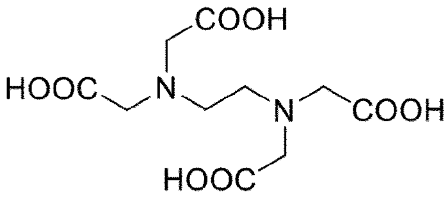

Fig. 2

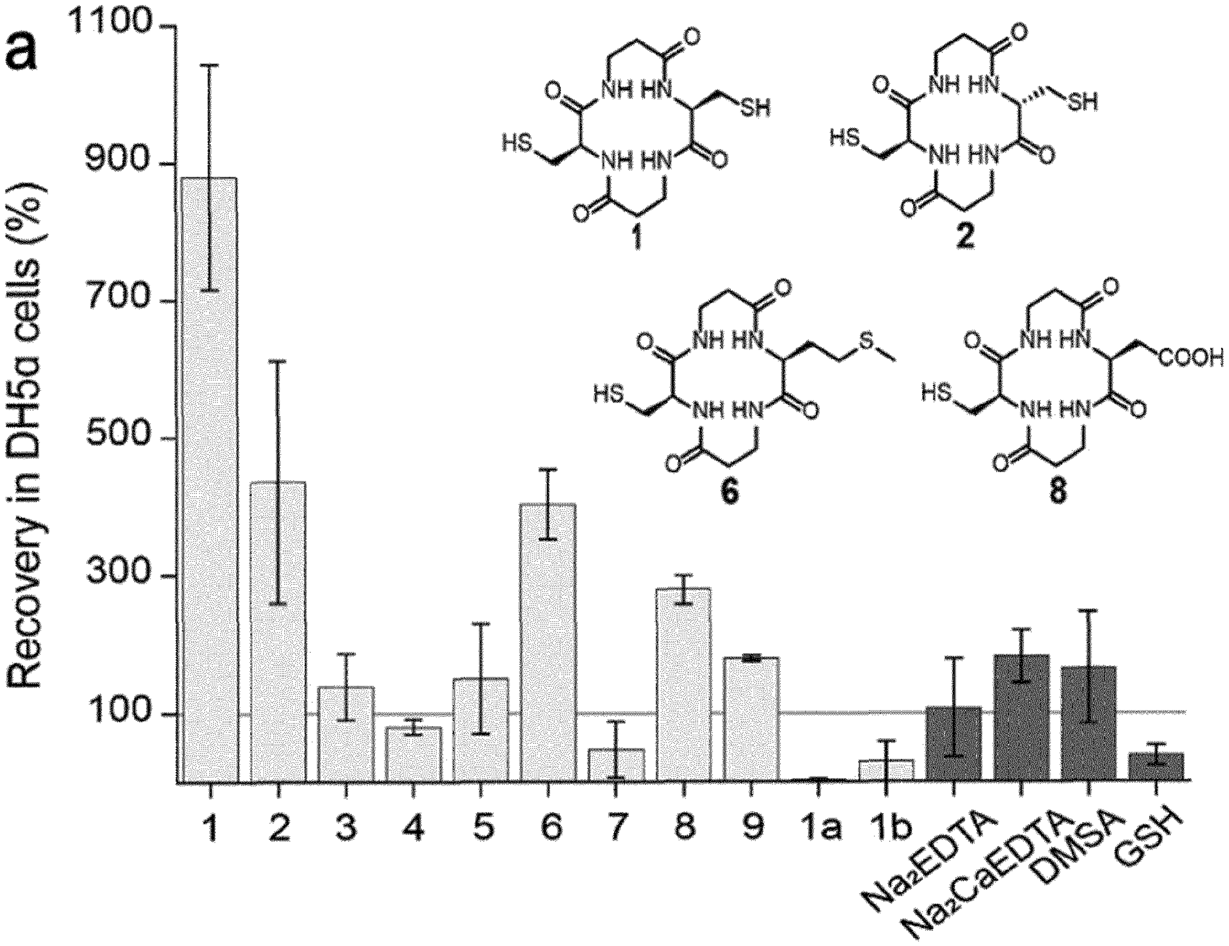
a
Recovery in DH5α cells (%)
1100
900
700
500
300
100
1
2
6
8
1
2
3
4
5
6
7
8
9
1a
1b
Na₂EDTA
Na₂CaEDTA
DMSA
GSH

Fig. 2 (continued)

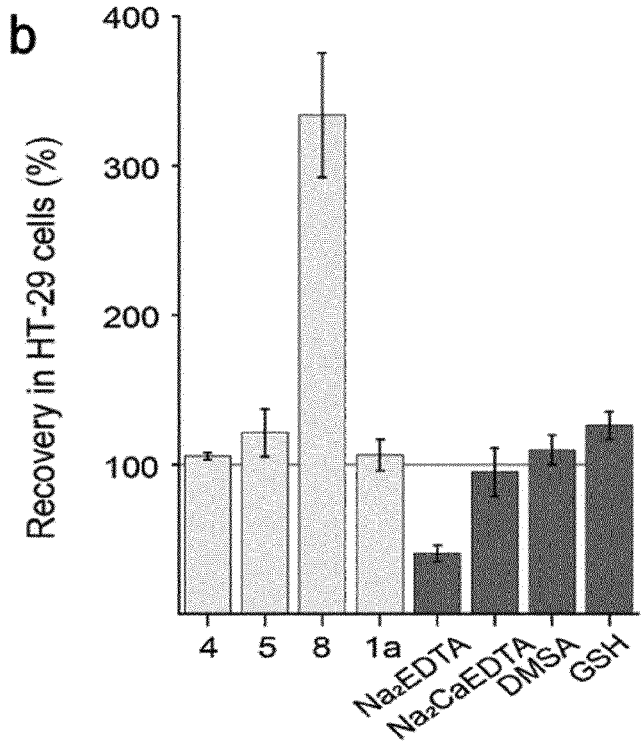


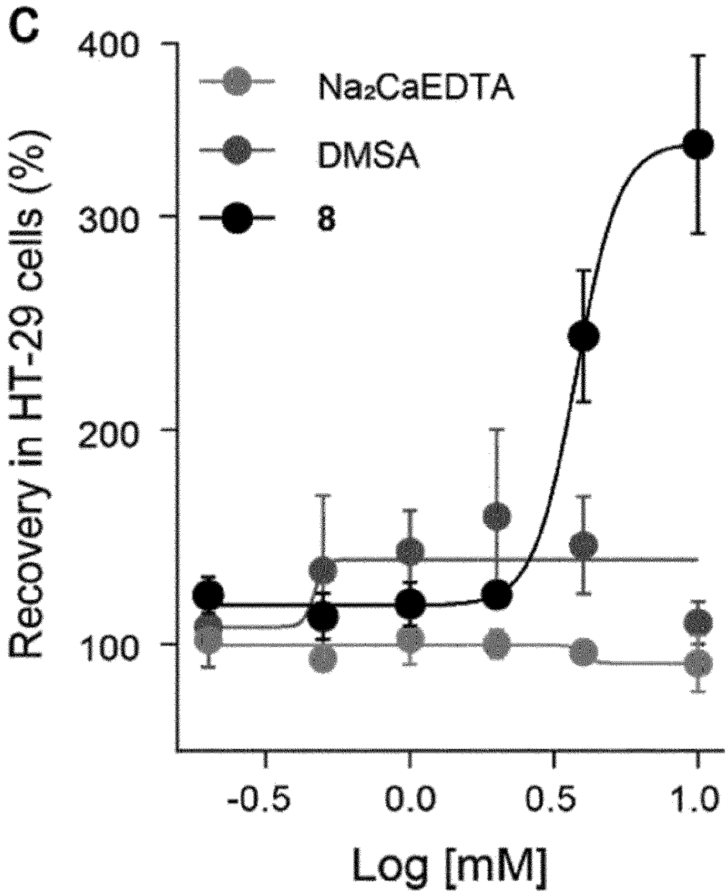

Fig. 2 (continued)

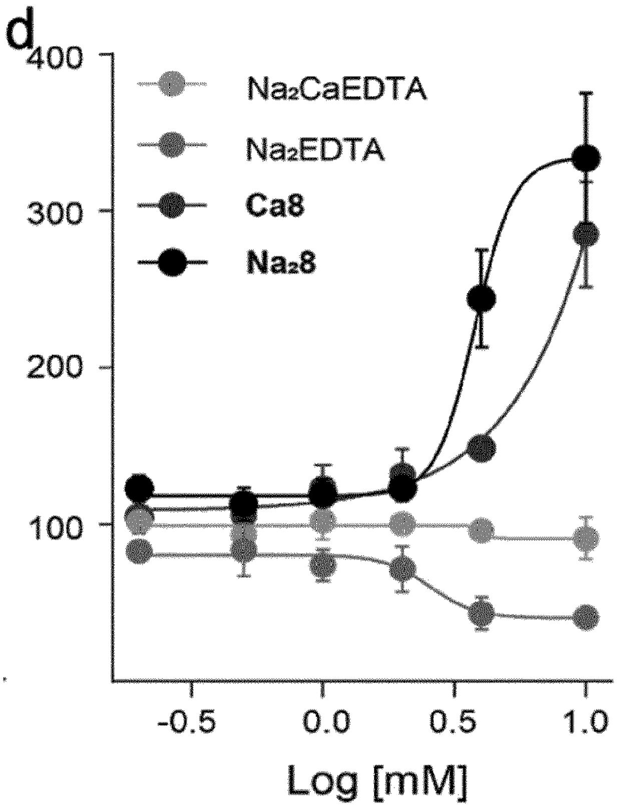


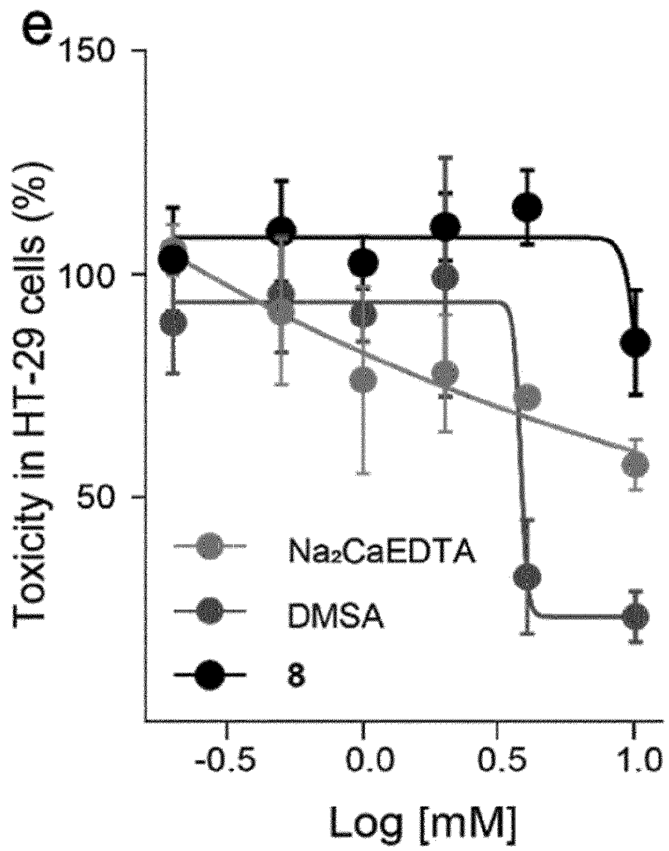

Fig. 3a

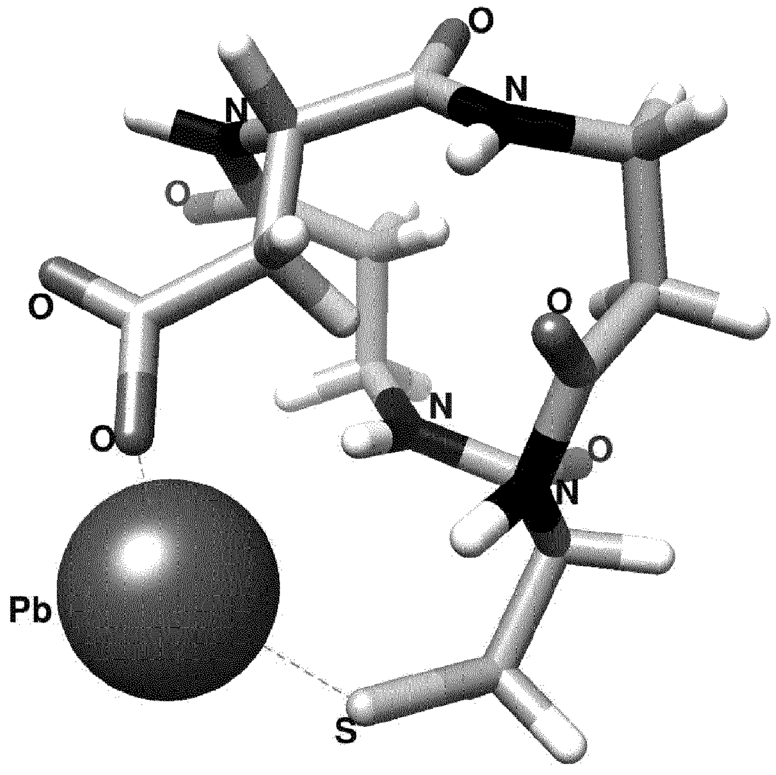

Fig. 3b

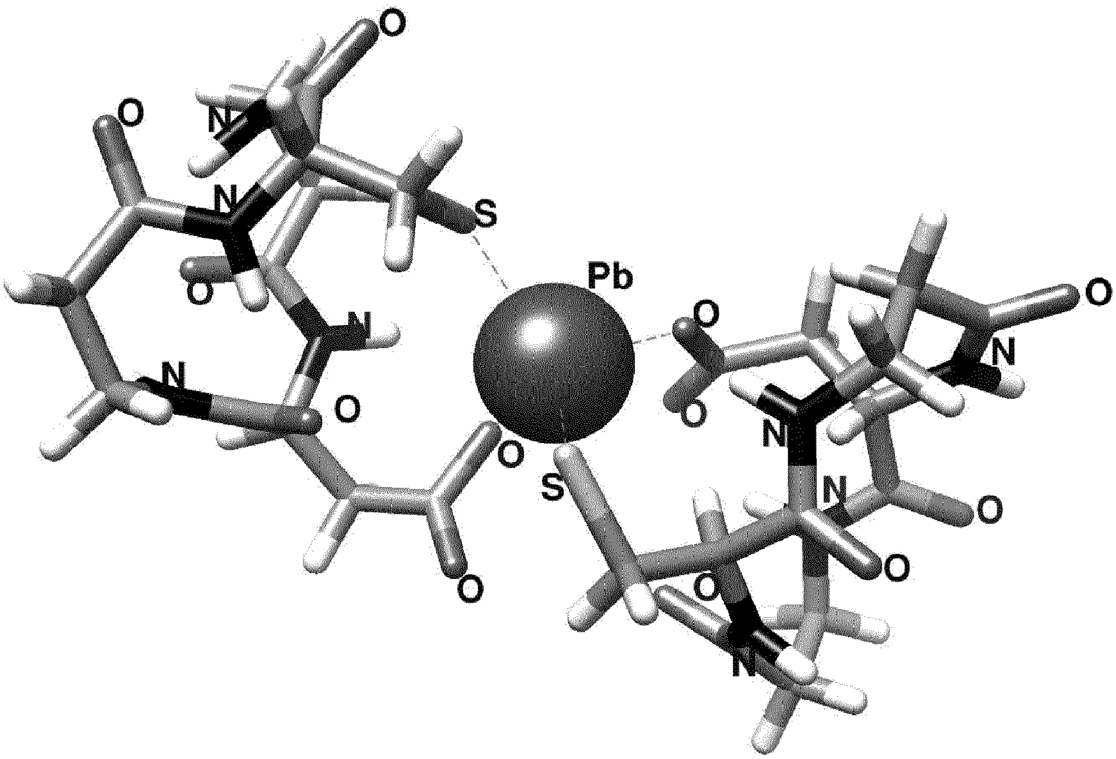

Europäisches Patentamt
European Patent Office
Office européen des brevets

# EUROPEAN SEARCH REPORT

Application Number
EP 20 21 1457

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BRASUN J ET AL: "Impact of ring size on the copper(II) coordination abilities of cyclic tetrapeptides", JOURNAL OF INORGANIC BIOCHEMISTRY, ELSEVIER INC, US, vol. 103, no. 5, 1 May 2009 (2009-05-01), pages 813-817, XP026052707, ISSN: 0162-0134, DOI: 10.1016/J.JINORGBIO.2009.02.003 [retrieved on 2009-02-23] | 1-3,5-8, 10-13 | INV. C07D257/02 A61K31/395 A61K31/4178 A61K31/555 A61P43/00 C07D403/06 C07D403/14 C07F7/24 C07K5/10 |
| Y | * figure 1 * * scheme 1 * | 15 | |
| X | SARFATI ROGER ET AL: "No 47. - Synthèse de diaza et têtraazacycloalcanes, intermédiaires, dans la synthèse de l'homaline. [Synthesis of diaza- and tetraazacycloalkanes, intermediates in the synthesis of homaline]", BULLETIN DE LA SOCIETE CHIMIQUE DE FRANCE, SOCIETY FRANCAISE DE CHIMIE , PARIS, FRANCE, no. 1, 1 January 1971 (1971-01-01), pages 255-263, XP009527498, ISSN: 0037-8968 * compound 2b * | 1-9,11 | |
| A | WO 01/77102 A1 (DU PONT PHARM CO [US]) 18 October 2001 (2001-10-18) * claims 1, 39 * | 1-14 | |
| Y | WO 2018/174337 A1 (FNG RES CO LTD [KR]; GOO YOUNG SAM [KR]; SON KI NAM [KR]) 27 September 2018 (2018-09-27) | 15 | |
| A | * claim 1 * | 1-13 | |

TECHNICAL FIELDS SEARCHED (IPC)

C07D
A61K
C07F
A61P
C07K

The present search report has been drawn up for all claims

2

| Place of search | Date of completion of the search | Examiner |
|---|---|---|
| Munich | 20 May 2021 | Fanni, Stefano |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 20 21 1457

This annex lists the patent family members relating to the patent documents cited in the above-mentioned European search report.
The members are as contained in the European Patent Office EDP file on
The European Patent Office is in no way liable for these particulars which are merely given for the purpose of information.

20-05-2021

| Patent document cited in search report | Publication date | Patent family member(s) | Publication date |
| --- | --- | --- | --- |
| WO 0177102 A1 | 18-10-2001 | AU 5144101 A | 23-10-2001 |
| | | US 2002004032 A1 | 10-01-2002 |
| | | WO 0177102 A1 | 18-10-2001 |
| WO 2018174337 A1 | 27-09-2018 | CN 110582557 A | 17-12-2019 |
| | | EP 3538631 A1 | 18-09-2019 |
| | | JP 2020518712 A | 25-06-2020 |
| | | KR 101851979 B1 | 07-06-2018 |
| | | US 2020002649 A1 | 02-01-2020 |
| | | WO 2018174337 A1 | 27-09-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **SAMBROOK et al.** Molecular Cloning: A Laboratory Manual. Cold Spring Harbor Laboratory Press, 2012
- **AUSUBEL et al.** Short Protocols in Molecular Biology. John Wiley & Sons, Inc, 2002
- *CHEMICAL ABSTRACTS,* 148893-10-1
- *CHEMICAL ABSTRACTS,* 330645-87-9
- *CHEMICAL ABSTRACTS,* 2592-95-2
- *CHEMICAL ABSTRACTS,* 693-13-0
- *CHEMICAL ABSTRACTS,* 538-75-0
- *CHEMICAL ABSTRACTS,* 26386-88-9
- *CHEMICAL ABSTRACTS,* 128625-52-5
- *CHEMICAL ABSTRACTS,* 7087-68-5
- Topical and Transdermal Drug Delivery: Principles and Practice. Wiley, 2011
- **GUY ; HANDCRAFT.** Transdermal Drug Delivery Systems: Revised and Expanded. CRC Press, 2002
- Topical Drug Delivery Formulations. CRC Press, 1989
- **L. LACHMAN et al.** The Theory and Practice of Industrial Pharmacy. 2013
- **SEARS, MARGARET E.** Chelation: Harnessing and Enhancing Heavy Metal Detoxification - A Review. *The Scientific World Journal,* 2013, vol. 2013, 13
- **WHITE, C. J. ; YUDIN, A. K.** Contemporary Strategies for Peptide Macrocyclization. *Nat. Chem.,* 2011, vol. 3 (7), 509-524, https://doi.org/10.1038/nchem.1062
- **FEOKTISTOVA, M. ; GESERICK, P. ; LEVERKUS, M.** Crystal Violet Assay for Determining Viability of Cultured Cells. *Cold Spring Harb. Protoc.,* 2016, vol. 2016 (4), 343-346, https://doi.org/10.1101/pdb.prot087379